# EUROPEAN PATENT SPECIFICATION

(11) **EP 2 867 669 B1**
(45) Date of publication and mention of the grant of the patent: **21.12.2016**
(21) Application number: 13731813.5
(22) Date of filing: 27.06.2013
(51) Int. Cl.: G01N 33/50

(54) **Methods for identifying diabetes drugs**
Verfahren zur Identifizierung von Diabetes-Arzneimitteln
Procédés pour l'identification de médicaments contre le diabète

(30) Priority: 27.06.2012 EP 12173884; 27.06.2012 US 201261664784 P; 15.02.2013 EP 13155457; 15.02.2013 US 201361765064 P
(43) Date of publication of application: 06.05.2015
(73) Proprietor: metanomics Health GmbH, 10589 Berlin (DE)
(72) Inventor: REIN, Dietrich, 10785 Berlin (DE); NIKIFOROVA, Victoria, 14542 Werder (DE); PADBERG, Inken, 10437 Berlin (DE); LIEBENBERG, Volker, 10557 Berlin (DE)
(74) Representative: Herzog, Fiesser & Partner Patentanwälte PartG mbB
(86) International application number: PCT/EP2013/063509
(87) International publication number: WO 2014/001451

(56) References cited:
- WO-A2-2004/112726
- WO-A2-2007/110357
- JP-A- 2008 228 646
- H. ZHANG ET AL: "Rosiglitazone reduces renal and plasma markers of oxidative injury and reverses urinary metabolite abnormalities in the amelioration of diabetic nephropathy", AJP: RENAL PHYSIOLOGY, vol. 295, no. 4, 30 July 2008 (2008-07-30) , pages F1071-F1081, XP055042369, ISSN: 0363-6127, DOI: 10.1152/ajprenal.90208.2008
- Dietrich Rein ET AL: "Metabolite Profiling Identifies Early Diabetes Changes; Leveraging Marker Candidates for Therapeutic Developments", , 21 February 2012 (2012-02-21), pages 1-1, XP055072066, Retrieved from the Internet: URL:http://www.google.nl/url?sa=t&rct=j&q= &esrc=s&source=web&cd=1&ved=0CCwQFjAA&url= http%3A%2F%2Fwww.metanomics-health.de%2Fdo wnload.php%3Ffile%3Duploads%2Fdownloads%2F 20120221_mtxh_t2dm_biomarkers_congress_pos ter.pdf&ei=DvHoUbuROun-4QSI5IC4CQ&usg=AFQj CNFh7FLKAnk1CdIVicJmzfQZymWKxA&bvm=bv.4947 8099,d.bGE [retrieved on 2013-07-19]

## Description

The present invention relates to screening methods for diabetes drugs. In particular, the invention concerns a method for identifying a drug against diabetes comprising determining the amount of glyoxylate in a test sample of a non-human animal subject suffering from diabetes or AGEs-related diabetic complications wherein said test sample has been taken after the subject has been brought into contact with a compound suspected to be a drug against diabetes and comparing the determined amount to a reference amount for glyoxylate, whereby a compound being a drug against diabetes is identified. Further contemplated is an ex vivo method for identifying a drug against diabetes comprising contacting test cells which produce glyoxylate with a compound suspected to be a drug against diabetes for a time and under conditions which allow for said compound to interact with the cells and to affect glyoxylate production, determining the amount of glyoxylate produced in said cells, and comparing the determined amount to a reference amount for glyoxylate, whereby a compound being a drug against diabetes is identified. Finally, a method for the manufacture of a drug against diabetes is disclosed.

The prevalence of diabetes mellitus has reached about 6% in the industrialised world and will increase up to 366 million affected people in 2030 worldwide. The most fre-quently reason (type), (about 90 %) for diabetes in the world is accounted for type 2 diabetes, which has a multifactorial pathogenesis. The pathological sequence for type2 diabetes entails many elements. A genetic predisposition contributes to the risk of developing type 2 diabetes but it's role is currently poorly understood. Whether the diabetes phenotype then occurs is influenced by many environmental factors that share an ability to stress the glucose homeostasis system, either by causing or worsening insulin resistance or impairing insulin secretion. Of course many hormones are taking part in the regulation of glucose metabolism, but the key hormone is insulin. Normoglycaemia is maintained by the balanced interplay between insulin action and insulin secretion. Insulin is produced by the pancreatic β-cell which is able to regulate very fast to different glucose demands. The main reason for type 2 diabetes is an increasing insulin resistance. Therefore, insulin action normally decreases but initially the system is able to compensate this by an increasing β-cell function. At this time perhaps only an impaired fasting glucose or an impaired glucose tolerance in the OGTT could be measured. But over time the β-cell will be overstressed by increasing insulin resistance and glucose toxicity and a type 2 diabetes could be diagnosed.

Apart from direct medical problems by high or low blood sugar the main medical and socioeconomic burden of the disease is caused by the associated complications. The devastating complications of diabetes mellitus are mostly macrovascular and microvascular diseases like chronic renal failure, retinopathy, periphery and autonomic neuropathy or myocardial infarction. Therefore, cardiovascular morbidity in patients with type 2 diabetes is two to four times greater than that of non-diabetic people.

In light of this mechanism, therapy of diabetes is currently based on monitoring the blood sugar levels and reducing an elevated level of blood sugar into a normal level by administration of exogenous insulin. To this end, exogenous insulin is injected into the blood. Alternatively, glucose levels in the blood may be regulated by nutritional diets and the exclusion of life-style risk factors, such as smoking, lack of exercise, high cholesterol levels, and excess body weight.

The Expert Committee of the ADA (American Diabetes Association) recognized an intermediate group of subjects whose glucose levels, although not meeting criteria for diabetes, are nevertheless too high to be considered normal. This group is defined as having fasting plasma glucose (FPG) levels >100 mg/dl (5.6 mmol/l) but <126 mg/dl (7.0 mmol/l) or 2-h values in the oral glucose tolerance test (OGTT) of >140 mg/dl (7.8 mmol/l) but <200 mg/dl (11.1 mmol/l). Thus, the categories of FPG values are as follows:
- FPG <100 mg/dl (5.6 mmol/l) = normal fasting glucose;
- FPG 100-125 mg/dl (5.6-6.9 mmol/l) = IFG (impaired fasting glucose);
- FPG >126 mg/dl (7.0 mmol/l) = provisional diagnosis of diabetes (the diagnosis must be confirmed, as described below).

The corresponding categories when the OGTT is used are the following:
- 2-h postload glucose <140 mg/dl (7.8 mmol/l) = normal glucose tolerance
- 2-h postload glucose 140-199 mg/dl (7.8 -11.1 mmol/l) = IGT (impaired glucose tol-erance)
- 2-h postload glucose >200 mg/dl (11.1 mmol/l) = provisional diagnosis of diabetes (the diagnosis must be confirmed, as described below).

Diagnosis of Diabetes mellitus type 2: Symptoms of diabetes plus casual plasma glucose concentration >200 mg/dl (11.1 mmol/l). Casual is defined as any time of day without regard to time since last meal. The classic symptoms of diabetes include polyuria, polydipsia, and unexplained weight loss. Alternatively: 2. FPG >126 mg/dl (7.0 mmol/l). Fasting is defined as no caloric intake for at least 8 h. Alternatively: 3. 2-h postload glucose >200 mg/dl (11.1 mmol/l) during an OGTT. The test should be performed as described by WHO, using a glucose load containing the equivalent of 75 g anhydrous glucose dissolved in water.

In the absence of unequivocal hyperglycemia, these criteria should be confirmed by repeat testing on a different day. The third measure (OGTT) is not recommended for routine clinical use. (American Diabetes Association, Diagnosis and Classification of Diabetes Mellitus, Diabetes Care 2006) However, an increase in the blood sugar levels or a decrease in the available insulin are developments which are rather downstream events in the development and progression of diabetes. Metabolic biomarkers for diabetes have been recently reported (see WO2007/110357; WO2007/110358; WO2009/14639; and WO2010/114897). The role of the metabolic biomarkers as potential targets for the development of diabetes drugs remains, however, elusive.

Components of the endoplasmic reticulum stress response pathway, such as XBP-1 or CHOP-1, have been proposed as screening markers in WO2004/112726.

Dietrich Rein, et. al. (retrieved from the internet representing a poster presentation from Metanomics Health GmbH at the Biomarkers Congress in Manchester on 21-02-2012 entitled: "Metabolite profiling identifies early diabetes changes; leveraging marker candidates for therapeutic developments") attempts to unravel the changes in early diabetes by metabolite profiling. Some of said metabolites were markedly altered. An increase in glyoxylate was measured in early diabetic patients.

Alike in the microorganisms, fungi, plants and some invertebrates e.g. roundworms, where glyoxylate is a side product of the glyoxylate cycle, in higher animals glyoxylate is mainly a product of the enzymatic glycolate oxidation in peroxisomes. The pathway has been well characterized with regard to the extensive studies on primary hyperoxaluria, a severe disease caused by the abnormally increased oxalate production, for which glyoxylate is the sole known immediate precursor. Besides glycolate oxidation, glyoxylate is a metabolic product of hydroxyproline in mitochondria, derived in a considerable extent from collagen of consumed animal protein (Belostotsky 2010, Am J Hum Genetics 87, 392-399). Another possible endogenous source of glyoxylate is glyoxal, which could be enzymatically converted to oxalate in HepG2 cells (Knight 2010, Horm Metab Res 42: 868-873). Glyoxylale has been also shown as a product of glycine deamination catalyzed by glycine oxidase in the process of the aerobic oxidation of glycine (Ratner 1944, J. Biol. Chem. 152: 119-133.).

It's worth noting with regard to diabetes, that the majority of these glyoxylate precursors can be considered as non-distant products of glucose metabolism. The indirect evidence for glucose being converted to glyoxylate is provided by the experiments in which HepG2 cells established from a human hepatoma and retaining many hepatocyte-specific functions, including the capability to convert glyoxylate to oxalate (Baker 2004, Am J Physiol Cell Physiol 287: C1359-C1365) were able to metabolize 13C6-labeled glucose into 13C2-labeled glycolate and 13C2-labeled oxalate (Knight loc. cit.), thus metabolically linking glucose with glyoxylate (as the direct intermediate in the glycolate convertion to oxalate: glycolate -> glyoxylate -> oxalate) and reasoning the correlation between glucose and glyoxylate levels in human plasma.

The glyoxylate molecule is also produced as an oxidized residue in the carboxyl-terminal amidation of glycine-extended peptides (Prigge 2000, Cell. Mol. Life Sci. 57: 1236-1259). Carboxylterminal amidation is a common post-translational event responsible for the bioactivation of approximately half of all peptide hormones (Foster 2011, Tetrahedron: Asymmetry 22: 283-293), including neuroendocrine peptides involved into the gut/brain signaling, which manages the sensation of hunger and satiety. Diabetes-related α-amidated peptide hormones, such as e.g. glucagon-like peptide 1, amylin, obestatin, or gastrin, represent attractive candidates for the treatment of type 2 diabetes, and for some the industrial analogues are patented as antidiabetic drugs. In the reaction of bioactivation of peptide hormones by amidation, glyoxylate is released in 1:1 molar ratio, thus being indicative of the presence of bioactive peptide hormones. Besides the amidation of the glycine-extended peptides, glyoxylate production has been also reported upon the amidation of the N-acylglycines (Wilcox 1999, Biochemistry 38: 3235-3245), hippurate (N-benzoylglycine) (Katopodis 1990, Biochemistry 29: 4541-4548), and the bile acid glycine conjugates (King 2000, Archives of Biochemistry and Biophysics 374: 107-117).

In light of the complex biochemical processes the search for new anti-diabetic drugs is cumbersome. In particular, there is apparently no single target which needs to be addressed by drug screening approaches. However, it would be highly desired to affect some metabolic aspects of diabetes more directly, in particular such pathways which result in comorbidities such as diabetic nephropathy.

Accordingly, the technical problem underlying the present invention must be seen as the provision of screening methods for efficiently identifying diabetes drugs, preferably, in a high throughput approach. The technical problem is solved by the embodiments characterized in the claims and described herein below.

Therefore, the present invention relates to a method for identifying a drug against diabetes comprising:
(a)determining the amount of glyoxylate in a test sample of non-human animal subject suffering from diabetes or an AGEs-related diabetic complication wherein said test sample has been taken after the subject has been brought into contact with a compound suspected to be a drug against diabetes; and (b)comparing the amount determined in step (a) to a reference amount for glyoxylate, whereby a compound being a drug against diabetes is identified.

The method according to the present invention is in one embodiment an ex vivo method, i.e. a method which is carried out on isolated test samples. The method referred to in accordance with the present invention, furthermore, may essentially consist of the aforementioned steps or may include further steps. Further steps may relate to sample pre-treatment or evaluation of the compound investigated by the method of the invention. Preferred further evaluation steps are described elsewhere herein. The method may partially or entirely be assisted by automation. For example, step a) can be automated by robotic and automated reader devices. Step b) can be automated by suitable data processing devices, such as a computer, comprising a program code which when being executed carries out the comparison automatically. A reference in such a case will be provided as a stored reference, e.g., from a database.

The term "identifying" as used herein refers to allocating a property and, preferably the property of being a drug against diabetes, to a compound to be investigated by the method of the present invention. The term may also, preferably, include providing and/or isolating and/or formulating said drug. It will be understood that the method of the present invention aiming at identifying a drug may be used in screening approaches starting from a largely unknown pool of compounds, such as chemical libraries of small compounds, libraries of compounds of nature, antibody or aptamer libraries, or libraries of inhibitory RNAs, suspected to comprise compounds suitable as drugs against diabetes or for validation approaches, e.g., if certain compounds have already been suggested to be suitable as drugs against diabetes based on other assays. Accordingly, identifying as used herein may encompass further steps of characterizing the compound, in particular, if the compound is comprised in a pool of compounds. Such further steps, preferably, include structural characterization, e.g., by applying mass spectroscopy, NMR spectroscopy and/or crystallographic X-ray diffraction pattern analysis and/or other techniques referred to elsewhere herein. Moreover, further steps included may also, preferably, aim at determining certain biochemical and/or physical properties of the compound. Further, steps which aim at investigating pharmacological properties may also be comprised by the term.

The term "drug against diabetes" as used herein refers to a drug effective in treating diabetes, i.e. in curing and/or ameliorating the disease or at least one symptom associated therewith or in ameliorating or preventing at least one side effect or comorbidity associated with the disease. Preferably, said comorbidity is diabetic nephropathy. Moreover, the term also encompasses all stages of candidate drugs from lead compounds for drug development up to validated compounds to be assessed for safety, efficacy or side effects or their potential to affect comorbidities. Accordingly, it will be understood that the method of the invention must not necessarily identify a compound which will mature into a drug but rather may merely identify a lead compound or candidate drug which may need further investigation in order to become a drug.

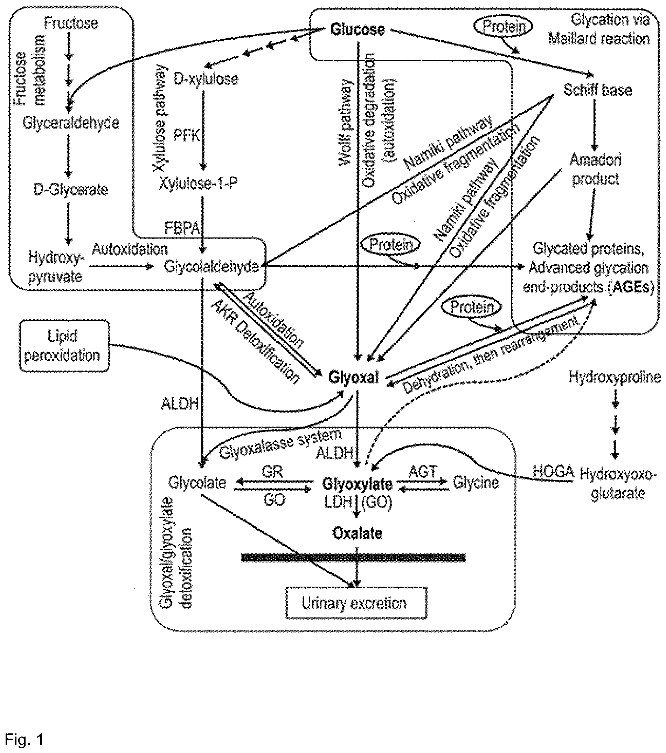

As a "compound suspected to be a drug against diabetes" any chemical compound could be applied in the method of the invention provided that the compound can be applied in non-toxic dosages and is, in principle, bioavailable such that it can influence the glyoxylate metabolism either directly or indirectly. A direct influence may be exert by a compound which interacts either with glyoxylate, e.g., by scavenging it, or with any metabolic precursor thereof or any enzyme or regulator involved in the glyoxylate metabolism in the subject. An indirect effect may be exerted by a compound which influences transcription or translation of such enzymes or regulators. Preferably, enzymes of the glyoxylate metabolism are those shown in Fig. 1, below, or catalysing an enzymatic conversion shown in Fig. 1. Preferably, enzymes envisaged in accordance with the present invention are glyoxylate reductase/hydroxypyruvate reductase (GRHPR), alanine:glyoxylate aminotransferase (AGT), aldehyde dehydrogenase (ALDH), glycolate oxidase (GO), glyoxylate reductase (GR), 4-hydroxy-2-oxoglutarate aldoase (HOGA), and/or lactate dehydrogenase (LDH). Ennzymatic conversions, influence on glyoxylate metabolism and disease associations are indicated in Table 1, below.

| Table 1. Genes which may be targeted to decrease glyoxylate level | | | | |
|---|---|---|---|---|
| Enzyme name | | Reaction | Involvement of glyoxylate... | Diseases associated with mutation/deficiency |
| AGT | alanine:glyoxylate aminotransferase | glyoxylate --> glycine | as a substrate; up-regulation may decrease glyoxylate | primary hyperoxaluria type 1 (PH1) |
| ALDH | aldehyde dehydrogenase | glyoxal --> glyoxylate | as a product; down-regulation may decrease glyoxylate | |
| GO | glycolate oxidase | glycolate --> glyoxylate | as a product; down-regulation may decrease glyoxylate | |
| GR | glyoxylate reductase | glyoxylate --> glycolate | as a substrate; up-regulation may decrease glyoxylate | primary hyperoxaluria type 2 (PH2) |
| HOGA | 4-hydroxy-2-oxoglutarate aldoase | hydroxyoxoglutarate --> glyoxylate | as a product; down-regulation may decrease glyoxylate | primary hyperoxaluria type 3 (PH3) |
| LDH | lactate dehydrogenase | glyoxylate --> oxalate | as a substrate; up-regulation may decrease glyoxylate | |

A regulator of the glyoxylate metabolism as referred to herein is, preferably, a transcription factor or signalling molecule that controls expression or activity of an enzyme of the glyoxylate metabolism. Preferably, regulators envisaged in accordance with the present invention are peroxisome proliferator-activated receptors (PPARs). Accordingly, compounds suspected to be drugs against diabetes may be selected from a plurality of different compound classes including small chemicals, chemical polymers, inorganic compounds, molecules or complexes or naturally occurring or artificial biomolecules, such as antibodies, aptamers, or nucleic acids.

The term "antibody" as used herein encompass to all types of antibodies which, preferably, specifically bind to glyoxylate or an enzyme or regulator of its metabolism. Preferably, the antibody is a monoclonal antibody, a polyclonal antibody, a single chain antibody, a chimeric antibody or any fragment or derivative of such antibodies being still capable of binding to glyoxylate or an enzyme or regulator of its metabolism. Moreover, the antibody shall, preferably, inhibit at least one of the biological or chemical activities of glyoxylate such as its activity during advanced glycation end product formation or an enzyme or regulator of the glyoxylate metabolism. Fragments and derivatives comprised by the term antibody as used herein encompass a bispecific antibody, a synthetic antibody, an Fab, F(ab)₂ Fv or scFv fragment, or a chemically modified derivative of any of these antibodies. Specific binding as used in the context of the antibody means that the antibody does not cross react with other components in the subject. Specific binding can be tested by various well known techniques. Antibodies or fragments thereof, in general, can be obtained by using methods which are described, e.g., in Harlow and Lane "Antibodies, A Laboratory Manual", CSH Press, Cold Spring Harbor, 1988. Monoclonal antibodies can be prepared by the techniques which comprise the fusion of mouse myeloma cells to spleen cells derived from immunized mammals and, preferably, immunized mice (Köhler 1975, Nature 256, 495, and Galfré 1981, Meth. Enzymol. 73,3). The term "aptamer" as used herein refers to oligonucleic acid or peptide molecules that bind to a specific target molecules (Ellington 1990, Nature 346 (6287): 818-22). Bock 1992, Nature 355 (6360): 564-6). Oligonucleic acid aptamers are engineered through repeated rounds of selection or the so called systematic evolution of ligands by exponential enrichment (SELEX technology). Peptide aptamers usually comprise of a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and capacity properties, such as thioredoxin-A. Peptide aptamer selection can be made using different systems including, e.g., the yeast two-hybrid system (see e.g., Hoppe-Seyler 2000, J Mol Med. 78 (8): 426-30). Moreover, the aptamer shall, preferably, inhibit at least one of the biological or chemical activities of glyoxylate such as its activity during advanced glycation end product formation or an enzyme or regulator of the glyoxylate metabolism.

The term "small molecule chemical compound" as used herein refers to a chemical compound that specifically interacts with glyoxylate or with an enzyme or regulator of its metabolism. A small molecule as used herein preferably has a molecular weight of less than 1000 Da, more preferably, less than 800 Da, less than 500 Da, less than 300 Da, or less than 200 Da. Such small molecules are capable of diffusing across cell membranes so that they can enter and reach intracellular sites of action. Suitable chemical compounds encompass small organic molecules. Suitable classes for such small organic molecules may, preferably, be isoprenoids, steroids, flavonoids, glycosides, alkaloids, lipids, alcohols, phenazines, phenols, polyketides, terpenes, or tetrapyrroles. Moreover, the small molecule chemical compound shall, preferably, inhibit at least one of the biological or chemical activities of glyoxylate such as its activity during advanced glycation end product formation or an enzyme or regulator of the glyoxylate metabolism. Preferably, the small molecule chemical compound is capable of scavenging glyoxylate similar to aminoguanidine or probably biguanide metformin. Moreover, preferably, the small molecule chemical compound is an antagonist of an enzyme of the glyoxylate biosynthesis.

The term "chemical polymer" as used herein refers to non-biological polymers, i.e. all polymeric molecules except of nucleic acids and peptides or proteins. The polymer shall, preferably, inhibit at least one of the biological or chemical activities of glyoxylate such as its activity during advanced glycation end product formation or an enzyme or regulator of the glyoxylate metabolism.

The term "inorganic compounds, molecules and complexes" encompasses elements, salts and other complexes of inorganic compounds or molecules. The said compounds, molecules or complexes shall, preferably, inhibit at least one of the biological or chemical activities of glyoxylate such as its activity during advanced glycation end product formation or an enzyme or regulator of the glyoxylate metabolism.

A nucleic acid to be used in accordance with the present invention as a compound to be investigated is, preferably, an inhibitory nucleic acid. Said inhibitory nucleic acids are, preferably, capable of interfering with transcription and/or translation of gene products being enzymes or regulators of the glyoxylate metabolism. Preferably, said inhibitory nucleic acids are capable of specifically binding to the transcription unit for such an enzyme or regulator and prevent transcription thereof upon binding or bind to the transcripts and prevent translation or facilitate degradation of the transcript. Such a nucleic acid is, preferably, selected from the group consisting of: an antisense RNA, a ribozyme, a siRNA, a micro RNA, a morpholino or a triple helix forming agent.

The term "antisense RNA" as used herein refers to RNA which comprises a nucleic acid sequence which is essentially or perfectly complementary to the target transcript of the enzyme or regulator of the glyoxylate metabolism. Preferably, an antisense nucleic acid molecule essentially consists of a nucleic acid sequence being complementary to at least 100 contiguous nucleotides, more preferably, at least 200, at least 300, at least 400 or at least 500 contiguous nucleotides of the target transcript. How to generate and use antisense nucleic acid molecules is well known in the art (see, e.g., Weiss, B. (ed.): Antisense Oligodeoxynucleotides and Antisense RNA : Novel Pharmacological and Therapeutic Agents, CRC Press, Boca Raton, FL, 1997).

The term "ribozyme" as used herein refers to catalytic RNA molecules possessing a well defined tertiary structure that allows for catalyzing either the hydrolysis of one of their own phosphodiester bonds (self-cleaving ribozymes), or the hydrolysis of bonds in other RNAs, but they have also been found to catalyze the aminotransferase activity of the ribosome. The ribozymes envisaged are, preferably, those which specifically hydrolyse the target transcripts of the enzyme or regulator of the glyoxylate metabolism. In particular, hammerhead ribozymes are preferably used. How to generate and use such ribozymes is well known in the art (see, e.g., Hean J, Weinberg MS (2008). "The Hammerhead Ribozyme Revisited: New Biological Insights for the Development of Therapeutic Agents and for Reverse Genomics Applications". In Morris KL. RNA and the Regulation of Gene Expression: A Hidden Layer of Complexity. Norfolk, England: Caister Academic Press).

The term "siRNA" as used herein refers to small interfering RNAs (siRNAs) which are complementary to target RNAs (encoding a gene of interest) and diminish or abolish gene expression by RNA interference (RNAi). Without being bound by theory, RNAi is generally used to silence expression of a gene of interest by targeting mRNA. Briefly, the process of RNAi in the cell is initiated by double stranded RNAs (dsRNAs) which are cleaved by a ribonuclease, thus producing siRNA duplexes. The siRNA binds to another intracellular enzyme complex which is thereby activated to target whatever mRNA molecules are homologous (or complementary) to the siRNA sequence. The function of the complex is to target the homologous mRNA molecule through base pairing interactions between one of the siRNA strands and the target mRNA. The mRNA is then cleaved approximately 12 nucleotides from the 3' terminus of the siRNA and degraded. In this manner, specific mRNAs can be targeted and degraded, thereby resulting in a loss of protein expression from the targeted mRNA. A complementary nucleotide sequence as used herein refers to the region on the RNA strand that is complementary to an RNA transcript of a portion of the target gene. The term "dsRNA" refers to RNA having a duplex structure comprising two complementary and anti-parallel nucleic acid strands. Not all nucleotides of a dsRNA necessarily exhibit complete Watson-Crick base pairs; the two RNA strands may be substantially complementary. The RNA strands forming the dsRNA may have the same or a different number of nucleotides, with the maximum number of base pairs being the number of nucleotides in the shortest strand of the dsRNA. Preferably, the dsRNA is no more than 49, more preferably less than 25, and most preferably between 19 and 23, nucleotides in length. dsRNAs of this length are particularly efficient in inhibiting the expression of the target gene using RNAi techniques. dsRNAs are subsequently degraded by a ribonuclease enzyme into short interfering RNAs (siRNAs). The complementary regions of the siRNA allow sufficient hybridization of the siRNA to the target RNA and thus mediate RNAi. In mammalian cells, siRNAs are approximately 21-25 nucleotides in length. The siRNA sequence needs to be of sufficient length to bring the siRNA and target RNA together through complementary base-pairing interactions. The length of the siRNA is preferably greater than or equal to ten nucleotides and of sufficient length to stably interact with the target RNA; specifically 15-30 nucleotides; more specifically any integer between 15 and 30 nucleotides, most preferably 15, 16, 17, 18, 19, 20, 21, 22, 23, 24, 25, 26, 27, 28, 29, and 30. By "sufficient length" is meant an oligonucleotide of greater than or equal to 15 nucleotides that is of a length great enough to provide the intended function under the expected condition. By "stably interact" is meant interaction of the small interfering RNA with target nucleic acid (e.g., by forming hydrogen bonds with complementary nucleotides in the target under physiological conditions). Generally, such complementarity is 100% between the siRNA and the RNA target, but can be less if desired, preferably 91%, 92%, 93%, 94%, 95%, 96%, 97%, 98%, or 99%. For example, 19 bases out of 21 bases may be base-paired. In some instances, where selection between various allelic variants is desired, 100% complementary to the target gene is required in order to effectively discern the target sequence from the other allelic sequence. When selecting between allelic targets, choice of length is also an important factor because it is the other factor involved in the percent complementary and the ability to differentiate between allelic differences. Methods relating to the use of RNAi to silence genes in organisms, including C. elegans, Drosophila, plants, and mammals, are known in the art (see, for example, Fire 1998, Nature 391:806-811; Fire 1999, Trends Genet. 15, 358-363; Sharp 2001, Genes Dev. 15,485-490; Hammond 2001, Nature Rev. Genet. 2, 1110-1119; Tuschl 2001, Chem. Biochem. 2, 239-245; Hamilton 1999, Science 286, 950-952; Hammond 2000, Nature 404, 293-296; Zamore 2000, Cell 101, 25-33; Bernstein 2001, Nature 409, 363-366; Elbashir 2001, Genes Dev. 15, 188-200; WO 0129058; WO 09932619; and Elbashir 2001, Nature 411: 494-498).

The term "microRNA" as used herein refers to a self complementary single-stranded RNA which comprises a sense and an antisense strand linked via a hairpin structure. The microRNA comprises a strand which is complementary to an RNA targeting sequences comprised by a transcript to be down regulated. microRNAs are processed into smaller single stranded RNAs and, therefore, presumably also act via the RNAi mechanisms. How to design and to synthesise microRNAs which specifically degrade a transcript of interest is known in the art and described, e.g., in EP 1 504 126 A2 or Dimond 2010, Genetic Engineering & Biotechnology News 30 (6):1.

The term "morpholino" refers to a synthetic nucleic acid molecule having a length of 20 to 30 nucleotides, preferably, about 25 nucleotides. Morpholinos bind to complementary sequences of target transcripts by standard nucleic acid base-pairing. They have standard nucleic acid bases which are bound to morpholine rings instead of deoxyribose rings and linked through phosphorodiamidate groups instead of phosphates (see, e.g., Summerton 1997, Antisense & Nucleic Acid Drug Development 7* (3): 187-95). Due to replacement of anionic phosphates with the uncharged phosphorodiamidate groups eliminates ionization in the usual physiological pH range, so morpholinos in organisms or cells are uncharged molecules. The entire backbone of a morpholino is made from these modified subunits. Unlike inhibitory small RNA molecules, morpholinos do not degrade their target RNA molecules. Rather, they sterically block binding to a target sequence within a RNA and simply getting in the way of molecules that might otherwise interact with the RNA (see, e.g., Summerton 1999, Biochimica et Biophysica Acta 1489 (1): 141-58).

The term "triple helix forming agent" as used herein refers to oligonucleotides which are capable of forming a triple helix with DNA and, in particular, which interfere upon forming of the triple-helix with transcription initiation or elongation of a desired target gene of the enzyme or regulator of the glyoxylate metabolism in the case of the inhibitor described by the present invention. The design and manufacture of triple helix forming agents is well known in the art (see, e.g., Vasquez 2002, Quart Rev Biophys 35: 89-107).

The term "glyoxylate" as referred to herein refers to glyoxylate as naturally occurring or artificially generated derivatives thereof. Naturally occurring derivatives are derivatives which are obtained from glyoxylate by metabolic conversions. Artificially generated derivatives are generated from glyoxylate during the analysis carried out e.g., derivatives which are required for GC-MS analysis and the like. It will be understood that the derivatives referred to hereinabove shall reflect the amount of the metabolite found in a subject, i.e. the amount of a derivative determined from a sample of the subject shall correlate with the amount of the metabolite found in the subject at the time when the sample has been taken. The following designations are used synonymously for glyoxylate: glyoxylic acid, formylformate, formylformic acid, glyoxalate, oxalaldehydate, oxalaldehydic acid, oxoacetate, oxoacetatic acid, oxoethanoate, oxoethanoic acid, alpha-ketoacetate or alpha-ketoacetic acid.

The term "test sample" as used in accordance with the present invention relates to a biological sample comprising glyoxylate in representative amounts for the glyoxylate metabolism of the subject. Samples from biological sources (i.e. biological samples) usually comprise a plurality of metabolites. Preferred biological samples to be used in the method of the present invention are samples from body fluids, preferably, blood, plasma, serum, lymph, sudor, saliva, tears, sperm, vaginal fluid, faeces, urine or cerebrospinal fluid, or samples derived, e.g., by biopsy, from cells, tissues or organs. This also encompasses samples comprising subcellular compartments or organelles, such as the mitochondria, Golgi network or peroxisomes. Moreover, biological samples also encompass gaseous samples, such as volatiles of an organism. Biological samples are derived from a subject as specified elsewhere herein. Techniques for obtaining the aforementioned different types of biological samples are well known in the art. For exampie, blood samples may be obtained by blood taking while tissue or organ samples are to be obtained, e.g., by biopsy. Most preferably, the test sample referred to herein is a blood, plasma or serum sample.

In a preferred embodiment, the test sample is a sample comprising a body fluid, i.e., a body fluid is comprised in the test sample. In a further preferred embodiment, the test sample is a blood, plasma or serum sample of a mammalian animal, more preferably a non-human mammal. In another preferred embodiment, the sample is a tissue, organ, or whole organism sample of a non-mammalian animal, more preferably, of a member of the Protostomia, even more preferably of a member of the Nematoda, most preferably of the Rhabditidae, like, e.g., a nematode of the species Caenorhabditis elegans.

The aforementioned samples are, preferably, pre-treated before they are used for the method of the present invention. As described in more detail below, said pre-treatment may include treatments required to release or separate the compounds or to remove excessive material or waste. Suitable techniques comprise centrifugation, extraction, fractioning, purification and/or enrichment of compounds. Moreover, other pre-treatments are carried out in order to provide the compounds in a form or concentration suitable for compound analysis. For example, if gaschromatography coupled mass spectrometry is used in the method of the present invention, it will be required to derivatize the compounds prior to the said gas chromatography. Suitable and necessary pre-treatments depend on the means used for carrying out the method of the invention and are well known to the person skilled in the art. Pre-treated samples as described before are also comprised by the term "sample" as used in accordance with the present invention. The term "diabetes" or "diabetes mellitus" as used herein refers to disease conditions in which the glucose metabolism is impaired. Said impairment results in hyperglycaemia. According to the World Health Organisation (WHO), diabetes in humans can be subdivided into four classes. Type 1 diabetes is caused by a lack of insulin. Insulin is produced by the so called pancreatic islet cells. Said cells may be destroyed by an autoimmune reaction in Type 1 diabetes (Type 1a). Moreover, Type 1 diabetes also encompasses an idiopathic variant (Type 1b). Type 2 diabetes is caused by an insulin resistance and relative insulin deficiency. Type 3 diabetes, according to the current classification, comprises all other specific types of diabetes mellitus. For example, the beta cells may have genetic defects affecting insulin production, insulin resistance may be caused genetically or the pancreas as such may be destroyed or impaired. Moreover, hormone deregulation or drugs may also cause Type 3 diabetes. Type 4 diabetes may occur during pregnancy. Preferably, diabetes as used herein refers to diabetes Type 2. (ADA criteria above) Further preferred diagnostic techniques are disclosed elsewhere in this specification. Further symptoms of diabetes are well known in the art and are described in the standard text books of medicine, such as Stedman or Pschyrembl.

A "diabetes-like condition" as referred herein refers to any metabolic disease or disorders which have an impairment of the gyloxylate metabolism comparable to the impairment which occurs in human diabetes mellitus patients. Such a condition may be the result of inbreeding, genetic engineering or may be chemically induced. Suitable diabetes or diabetes-like condition disease models are described elsewhere herein in detail. Preferably, the impairment of glyoxylate metabolism referred to herein is associated with an accumulation of advanced glycation end products (AGEs). Accordingly, a preferred diabetes-like condition is an accumulation of AGEs. Such an accumulation has also reported to correlate to life-span in certain organism such as nematodes and, in particular, in the nematode and model organism *C. elegans.*

The term "subject" as used herein refers to a test or laboratory non-human animal. Preferably, said animal suffers from diabetes mellitus or a diabetes-like condition as referred to herein above. Accordingly, said subject shall, in principle, be able to develop said disease or condition. More preferably, diabetes or diabetes-like condition may be developed endogenously, e.g. as a consequence of a higher prevalence for diabetes or diabetes-like conditions in certain inbred lines of subjects referred to herein, or as a result of an exogenous stimulus. It will be understood that the subjects from which the test samples investigated by the method of the present invention are obtained are laboratory animals which will be killed. Preferably, the subject, thus, is a rodent, preferably a mouse, a rat or a guinea pig, a pig, a rabbit, a cat, a dog, a goat, a sheep, a cow, a horse, a non-human primate, such as a baboon, a monkey, or a chimpanzee, a dolphin, or a nematode.

Diabetic models have been established for pigs, and some other non-human animals. Such animals could be used as subjects in the method of the present invention. In particular, the models are based on transgenic animals expressing the AMP-activated protein kinase (AMPK) y3 subunit, in particular, expressing the Prkag3 gene in skeletal muscle. These animals can be used as a model for diseases relating to energy metabolism, including type 2 diabetes. AMPK is a major cellular regulator of lipid and glucose metabolism, and as such has a key role in regulating the energy metabolism in eukaryotic cells. Activated AMPK turns on ATP-producing pathways and inhibits ATP-consuming pathways. AMPK also can inactivate glycogen synthase, the key regulatory enzyme of glycogen synthesis, it is recognized as a major regulator of lipid biosynthetic pathways, and has a wider role in metabolic regulation. Generally, an increase in overall activity of AMPK in muscle increases cellular energy levels by inhibiting anabolic energy consuming pathways (fatty acid synthesis, protein synthesis, etc.) and stimulating energy producing, catabolic pathways (fatty acid oxidation, glucose transport, etc.), which is coupled to increased glucose uptake and lowered blood glucose levels; see US2005/172348.

Moreover, a transgenic pig model has been reported wherein the pigs express a dominant-negative glucose-dependent insufinotropic polypeptide-1 receptor. Such pigs develop glucose intolerance and a reduced proliferation of pancreatic beta cells resembling those found in type 2 diabetes. Such transgenic pigs can also, preferably, be applied as subjects in the method of the invention; see Renner 2010, Diabetes 59: 1228-1238.

Further, an obesity-resistant transgenic pig expressing a leptin gene or an adiponectin gene has been generated which could be used as a subject for the method of the present invention; see JP2006-121964.

Moreover, diet-induced pigs with obesity/leptin resistance have been reported that may also be used as subjects for the method of the present invention. A pig breed with leptin resistance and predisposition to obesity (the Iberian pig) can be used as a robust, amenable, and reliable translational model for studies on metabolic syndrome and type 2 diabetes. Special feeding of the Iberian pigs during three months with ad libitum access to food enriched with saturated fat (SFAD group; food consumption 4.5 kg/animal/day) induces development in the animals of central obesity, dyslipidemia, insulin resistance and impaired glucose tolerance, and elevated blood pressure; the five parameters associated with the metabolic syndrome, see Torres-Rovira L, Astiz S, Caro A, Lopez-Bote C, Ovilo C, Pallares P, Perez-Solana ML, Sanchez-Sanchez R, Gonzalez-Bulnes A (2012) Diet-induced Swine model with obesity/leptin resistance for the study of metabolic syndrome and type 2 diabetes. Scientific World Journal. 2012; 2012:5101.

Furthermore, the so called "Ossabaw" pig as a model of metabolic syndrome may be used as a subject for the method of the present invention. The Ossabaw pig is used as a model of metabolic syndrome (MetS) because of its thrifty genotype. Ossabaw pigs develop features of MetS, when fed high-fat diet. To induce MetS, high-fat, high-cholesterol atherogenic calorie-matched diet has to be used for 40 weeks. As a result of this diet, MetS characteristics including obesity, glucose intolerance, hyperinsulinemia, and elevated arterial pressure are elevated. In pigs demonstrating MetS increased fasting blood glucose is increased on the order of 1.4-2.2-fold, while in humans an approximate 1.4-fold increase in the fasting blood glucose level renders a diagnosis of diabetes mellitus. Although similar to humans, glucose homeostasis in pig shows important differences that should be taken into careful consideration when defining diabetes in pig. Chief among these concerns are the lower fasting glucose levels (60 to 80 mg/dL) in healthy pig compared with humans (approximately 90 mg/dL). Also of importance is that pigs have relatively high glucose tolerance to oral glucose load and increased clearance after intravenous glucose load. In addition, pancreatic β-cell:body mass ratio in pig is twice that in humans, suggesting considerable insulin secretory reserve in pig. Taken together, these observations suggest that lower thresholds for the diagnosis of prediabetes and diabetes mellitus should be considered for pig. Ossabaw pig remarkably mimic the MetS seen in humans and can serve as an excellent large animal model for the study of metabolic abnormalities, see Neeb ZP, Edwards JM, Alloosh M, Long X, Mokelke EA, Sturek M (2010) Metabolic syndrome and coronary artery disease in Ossabaw compared with Yucatan swine. Comp Med. 60(4): 300-15.

In mice, diabetes can be induced by chemicals and mice and rats harbouring such a chemically-induced diabetes can also be used in the method of the present invention. Streptozotocin (STZ) is an antibiotic that can cause pancreatic β-cell destruction, so it is widely used experimentally as an agent capable of inducing insulin-dependent diabetes mellitus (IDDM), also known as type 1 diabetes mellitus (T1 DM). The protocols are developed for the production of insulin deficiency and hyperglycemia in mice and rats, using STZ. These models for diabetes can be employed for assessing the mechanisms of T1 DM, screening potential therapies for the treatment of this condition, and evaluation of therapeutic options. The T1 DM can be induced in mouse through the injection with streptozotocin. Several genotype lines can be used for such induction: a C57BL/6J mice, Swiss albino mice, BALB/c mice. Streptozotocin (STZ) is toxic to the insulin-producing beta cells in the pancreas, thereby causing type I diabetes. To establish the STZ-induced diabetes state, 8-10 weeks old mice are fasted overnight, and treated with freshly prepared STZ solution at a dose of 130 mg/kg body weight for C57BL/6J mice or 180 mg/kg body weight for Swiss albino mice via intraperitoneal injection. C57BL/6J mice that are not injected with STZ, or Swiss albino mice injected with equivalent amount citrate buffer serve as corresponding controls. Four hours after injection, the mice receive 200 µl of 20% glucose via intra-peritoneal injection to prevent hypoglycemia caused by sudden release of large amounts of insulin into the blood stream due to STZ-induced β-cell destruction. Mice can be fed a normal chow diet (ssniff R/M-H Autoklavierbar. www.ssniff.de). The blood glucose levels of each mouse are measured daily following 4-6 hours of fasting. The benefits of the STZ-induced diabetic mouse model include that it allows one to induce diabetes in genetically altered mice, maintain mice in a controlled environment, regularly monitor and directly measure serum and bone factors, obtain bone samples for high-resolution analyses, and chose the time of diabetic induction; see Kenneth K. Wu, Youming Huan (2008) Streptozotocin-induced diabetic models in mice and rats. Curr. Protoc. Pharmacol. 40:5.47.1-5.47.14, Zhang Y, Zhang Y, Bone RN, Cui W, Peng JB, Siegal GP, Wang H, Wu H (2012) Regeneration of Pancreatic Non-β Endocrine Cells in Adult Mice following a Single Diabetes-Inducing Dose of Streptozotocin. PLoS One. 2012;7(5):e36675, Arora A, Ojha SK, Vohora D (2009) Characterization of streptozotocin induced Diabetes mellitus in Swiss albino mice. Global J Pharmacol 3(2): 81-84, and Motyl K, McCabe LR (2009) Streptozotocin, type I diabetes severity and bone. Biological Procedures Online 11: 296-315.

Moreover, there are mutant mouse models for type 2 diabetes, i.e. the obese db/db^{-/-} mouse, which has a mutation in the receptor for the hormone leptin, and the ob/ob^{-/-} mouse with defective leptin pathway and severe obesity. The db gene encodes for a G-to-T point mutation in the receptor for the hormone leptin. The lean littermates, which possess one mutant and one normal copy of the leptin (dab-/+), are used as controls. Leptin functions as a satiety hormone and the absence of the leptin signalling pathway causes an excessive food intake and an obese phenotype in the db/db-/- mouse. In combination with the diabetes-sensitive black Kaliss background (C57BL/KsJ), these mice develop severe diabetes. In the C57BL/6J background, less hyperglycemia is found despite similar degrees of hyperphagia and weight gain. Another obese, diabetic mouse is the ob/ob^{-/-} mouse. This ob/ob-/- mouse differs from the db/db^{-/-} mouse in that it has a deficiency in the production of leptin but intact leptin signalling. Similar to the db/db^{-/-} mouse, the ob/ob^{-/-} mouse in the C57BLKS/J background develops β-cell atrophy and severe hyperglycemia, whereas ob/ob-/- mice in the C57BL/6J background develop only mild hyperglycemia. In the C57BLKS/J db/db-/- mouse, hyperinsulinemia is noted by 10 days of age and blood glucose levels are slightly elevated at 1 mo of age. After 1 mo of age, the db/db-/- mice are distinguished from wild-type and heterozygous mice by the presence of increased fat deposition in the inguinal and axillary regions. The db/db-/- mouse develops frank hyperglycemia by 8 wk of age. There is a progressive increase in food and water intake associated with progressive weight gain until 4-5 mo of age. Progressive hyperglycemia is noted with peak levels of glucose at 16 week of age. For the experimenting, three months old mice can be used, fed a normal chow diet (ssniff R/M-H Autoklavierbar. www.ssniff.de). Mice are provided with food and water ad libitum and maintained in a room with alternating twelve-hour light/dark cycle and kept at 22°C. For blood and tissue sampling, overnight-fasted mice are killed in the morning between 8:00 and 11:00 AM. Blood is obtained from the mandibular vein, and then mice are immediately killed by cervical dislocation. Blood is immediately centrifuged (3,000rpm at 4°C, for 5min) and plasma separated from the erythrocytes for the assay of glucose. The packed erythrocytes are used for the determination of glycosylated haemoglobin; see Lee SM, Bressler R (1981) Prevention of diabetic nephropathy by diet control in the db/db mouse. Diabetes 30:106-111, Sharma K, McCue P, Dunn SR (2003) Diabetic kidney disease in the db/db mouse. Renal Physiol 284(6): F1138-F1144, Broderick TL, Jankowski M, Wang D, Danalache BA, Parrott CR, Gutkowska J. (2012) Downregulation in GATA4 and Downstream Structural and Contractile Genes in the db/db Mouse Heart. ISRN Endocrinol. 2012;2012:736860, for details.

Moreover, inbred NOD (non-obese diabetic) mice or inbred BB rats have been reported to have an increased prevalence for diabetes and could, thus, also be used as subjects in the method of the present invention; see, e.g., Edward H. Leiter, Michal Prochazka, Douglas L. Coleman (1987): American Journal of Pathology. 128(2): 380-383.

Moreover, there is a nematode model for diabetes-like conditions available which could also be used as subject according to the invention. Growing of Caenorhabditis elegans on high glucose (100mM) allows achieving glucose concentrations resembling the hyperglycemic conditions in diabetic patients. C. elegans was used as a model for diabetes research to understand basic mechanisms underlying glucose effects on cellular and mitochondrial function (Vosseller,K: O-GlcNAc and aging: C. elegans as a genetic model to test O-GlcNAc roles in type II diabetic insulin resistance. Aging (Albany NY) 2:749-751, 2010; Mendler,M, Schlotterer,A, Morcos,M, Nawroth,PP: Understanding diabetic polyneuropathy and longevity: what can we learn from the nematode Caenorhabditis elegans? Exp Clin Endocrinol Diabetes 120:182-183, 2012). Nematodes are cultivated on nematode growth medium (NGM) agar and maintained at 20°C. Animals are maintained on living Escherichia coli (OP50) from a standardized overnight culture with an OD of 1.5, which is added to the surface of the NGM plates. Dead bacteria killed by sonication can also be used. To achieve a glucose concentration in a C. elegans whole body extract of 10-15 mmol/l, resembling the glucose concentrations in diabetic patients under poor glucose control, 150 µl of a 400 mmol/l glucose solution is used. 40 mmol/l glucose concentration in the agar results in 14 mmol/l glucose concentration in the C. elegans whole-body extract. C. elegans are kept for 5 days under high glucose, then harvested and washed. When total body glucose concentration reaches 14 mmol/l, significant effects on life span can be achieved. This in vivo model nematode provides a reliable tool to decipher changes in cellular functions induced by glucose concentrations that are within the range observed in poorly controlled diabetic patients, see Schlotterer A, Kukudov G, Bozorgmehr F, Hutter H, Du X, Oikonomou D, Ibrahim Y, Pfisterer F, Rabbani N, Thornalley P, Sayed A, Fleming T, Humpert P, Schwenger V, Zeier M, Hamann A, Stern D, Brownlee M, Bierhaus A, Nawroth P, Morcos M (2009) C. elegans as model for the study of high glucose-mediated life span reduction. Diabetes 58: 2450-2456, for details.

The term "determining" as used herein refers to determining at least one characteristic feature of glyoxylate comprised by the sample referred to herein. Characteristic features in accordance with the present invention are features which characterize the physical and/or chemical properties including biochemical properties of glyoxylate. Such properties include, e.g., molecular weight, viscosity, density, electrical charge, spin, optical activity, elementary composition, chemical structure, capability to react with other compounds, capability to elicit a response in a biological read out system and the like. Values for said properties may serve as characteristic features and can be determined by techniques well known in the art. Moreover, the characteristic feature may be any feature which is derived from the values of the physical and/or chemical properties of glyoxylate by standard operations, e.g., mathematical calculations such as multiplication, division or logarithmic calculus. Most preferably, the at least one characteristic feature allows the determination and/or chemical identification of glyoxylate.

Glyoxylate comprised by a test sample may be determined in accordance with the present invention quantitatively or qualitatively. For qualitative determination, the presence or absence of glyoxylate will be determined by a suitable technique. Moreover, qualitative determination may, preferably, include determination of the chemical structure or composition. For quantitative determination, either the precise amount of glyoxylate present in the sample will be determined or the relative amount thereof will be determined, preferably, based on the value determined for the characteristic feature(s) referred to herein above. The relative amount may be determined in a case were the precise amount glyoxylate can or shall not be determined. In said case, it can be determined whether the amount in which glyoxylate is present is enlarged or diminished with respect to a reference amount. Quantitatively analysing glyoxylate, thus, also includes what is sometimes referred to as semi-quantitative analysis.

Moreover, determining as used in the method according to the present invention, preferably, includes using a compound separation step prior to the analysis step referred to before. Preferably, said compound separation step yields a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado. Most preferably, LC and/or GC are chromatographic techniques to be envisaged by the method of the present invention. Suitable devices for such determination of metabolites, such as glyoxylate, are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Most preferably, LC-MS and/or GC-MS are used as described in detail below. Said techniques are disclosed in, e.g., Nissen, Journal of Chromatography A, 703, 1995: 37-57, US 4,540,884 or US 5,397,894. As an alternative or in addition to mass spectrometry techniques, the following techniques may be used for compound determination and quantification: nuclear magnetic resonance (NMR), magnetic resonance imaging (MRI), Fourier transform infrared analysis (FT-IR), ultra violet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado. The method of the present invention shall be, preferably, assisted by automation. For example, sample processing or pre-treatment can be automated by robotics. Data processing and comparison is, preferably, assisted by suitable computer programs and databases. Automation as described herein before allows using the method of the present invention in high-throughput approaches.

As described above, in a preferred embodiment of the method of the present invention, said determining of glyoxylate comprises mass spectrometry (MS). Mass spectrometry as used herein encompasses all techniques which allow for the determination of the molecular weight (i.e. the mass) or a mass variable corresponding to a compound, i.e. a metabolite, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approaches using the aforementioned techniques. How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis. Details on said most preferred mass spectrometry to be used in accordance with the present invention can be found in the Examples, below.

More preferably, said mass spectrometry is liquid chromatography (LC) MS and/or or even more preferred gas chromatography (GC) MS. Liquid chromatography as used herein refers to all techniques which allow for separation of compounds (i.e. metabolites including glyoxylate) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatised prior to gas chromatography. Suitable techniques for derivatisation are well known in the art. Preferably, derivatisation in accordance with the present invention relates to methoxymation and trimethylsilylation of, preferably, polar compounds and transmethylation, methoxymation and trimethylsilylation of, preferably, non-polar (i.e. lipophilic) compounds.

Moreover, glyoxylate can also be determined by a specific chemical or biological assay. Said assay shall comprise means which allow for specifically detecting glyoxylate in the sample. Preferably, said means are capable of specifically recognizing the chemical structure of glyoxylate or are capable of specifically identifying the glyoxylate based on its capability to react with other compounds or its capability to elicit a response in a biological read out system (e.g., induction of a reporter gene). Means which are capable of specifically recognizing the chemical structure of glyoxylate are detection agents for glyoxylate, preferably, antibodies, proteins or aptamers which specifically bind to gyloxylate. Specific antibodies, for instance, may be obtained using glyoxylate as antigen or from phage antibody libraries by methods well known in the art. Antibodies as referred to herein include both polyclonal and monoclonal antibodies, as well as fragments thereof, such as Fv, Fab and F(ab)₂ fragments that are capable of binding the antigen or hapten. Moreover, encompassed are single chain antibodies and all types of chimeric antibodies. Suitable proteins which are capable of specifically recognizing the glyoxylate are, preferably, enzymes which are involved in the metabolic conversion of the said metabolite. Said enzymes may either use glyoxylate as a substrate or may convert a substrate into the metabolite. Aptamers which specifically bind to glyoxylate can be generated by methods well known in the art (Ellington 1990, Nature 346:818-822; Vater 2003, Curr Opin Drug Discov Devel 6(2): 253-261). Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, glyoxylate may also be identified based on its capability to react with other compounds, i.e. by a specific chemical reaction. Further detection methods such as capillary electrophoresis (Hubert 2001, Clinical Chemistry 47: 1319-1321) and colorimetric methods (Kyaw 1978, Clin Chim Acta 86(2):153-7) can be used. Further, glyoxylate may be determined in a sample due to its capability to elicit a response in a biological read out system. The biological response shall be detected as read out indicating the presence and/or the amount of glyoxylate comprised by the sample. The biological response may be, e.g., the induction of gene expression or a phenotypic response of a cell or response may be, e.g., the induction of gene expression or a phenotypic response of a cell or an organism. Moreover, glyoxylate can be measured by enzymatic conversions where based on enzymatic conversion of glyoxylate redox equivalents are generated which can, in turn, be detected by a suitable readout system as described in US2010/0143897, King 2000, Archives of Biochemistry and Biophysics 374: 107-117. Other techniques for determining glyoxylate are based on a derivatization with a detectable moiety and subsequent determination of the detectable derivative (Baker 2004, Am J Physiol Cell Physiol 287: C1359-1365).

Further, it is to be understood that depending on the technique used for determining the glyoxylate, the analyte which will be detected could be a derivative of the physiologically occurring glyoxylate, i.e. the metabolite present within a subject. Such analytes may be generated as a result of sample preparation or detection means. The compounds referred to herein are deemed to be analytes. However, as set forth above, these analytes will represent glyoxylate in a qualitative and quantitative manner.

The term "reference amount for glyoxylate" relates to an amount which allows allocating a test amount as being either an amount associated with diabetes or a diabetes-like condition, or not. Accordingly, the reference amount may reflect the amount of glyoxylate which is normally found in subjects suffering from diabetes or a diabetes-like condition or the amount which is normally found in subjects not diabetes or a diabetes-like condition. Based on the identity or difference of a test amount compared to the reference amount, the test amount can be allocated to either reference group. Such a reference amount may be derived from a single subject or group of subjects. Preferably, a group of subjects comprises at least 5, at least 10, at least 15, at least 20, at least 25, at least 50, or at least 100 reference subjects. Moreover, a threshold may be used as reference amount as well. Such a threshold may be, preferably, the upper limit of normal for glyoxylate in a given animal species. Said upper limit of normal is the upper limit of physiological amounts of glyoxylate found in animals of said species. A test amount which is higher than the upper limit of normal is indicative for diabetes or a diabetes-like condition while a test amount which equals the upper limit of normal or which is below the upper limit of normal, shall be indicative for the absence of diabetes or a diabetes-like condition. It follows that if an amount for glyoxylate can be determined in a test sample which equals or is below the upper limit of normal upon wherein the said test sample has been taken after administration of a compound suspected to be drug against diabetes, the said compound shall be identified as being a drug against diabetes. In order to define such an upper limit of normal, preferably a cohort of apparently healthy subjects may be investigated. Suitable statistical tests for determining the size of such a cohort are well known to the skilled person.

In a preferred embodiment of the method of the present invention, the reference amount for glyoxylate is derived from a subject or group of subjects suffering from diabetes or an AGEs-related diabetic complication wherein said subject(s) have not been brought into contact with the compound suspected to be a drug against diabetes. More preferably, an identical or increased amount of glyoxylate in the test sample in comparison to the reference amount identifies a compound not being a drug against diabetes, whereas a decreased amount of glyoxylate in the test sample in comparison to the reference amount identifies a compound being a drug against diabetes.

In another preferred embodiment of the method of the present invention, said reference amount is the amount of glyoxylate present in a reference sample which has been taken prior to contacting the subject to the compound suspected to be a drug against diabetes. More preferably, an identical or increased amount of glyoxylate in the test sample in comparison to the reference amount identifies a compound not being a drug against diabetes, whereas a decreased amount of glyoxylate in the test sample in comparison to the reference amount identifies a compound being a drug against diabetes.

In yet another preferred embodiment of the method of the present invention, said reference amount for glyoxylate is derived from an apparently healthy subject or group of subjects with regard to diabetes or an AGEs-related diabetic complication. More preferably, an increased amount of glyoxylate in the test sample in comparison to the reference amount identifies a compound not being a drug against diabetes, whereas an identical or decreased amount of glyoxylate in the test sample in comparison to the reference amount identifies a compound being a drug against diabetes.

The term "comparing" refers to assessing whether the amount resulting from the quantitative determination of glyoxylate is identical to a reference amount or differs therefrom.

In case the reference amount for glyoxylate is derived from a subject or group of subjects suffering from diabetes or a diabetes-like condition wherein said subject(s) have not been brought into contact with the compound suspected to be a drug against diabetes or is from the subject prior to the treatment with the compound suspected to be a drug against diabetes, the drug can be identified based on the degree of differences between the amount obtained from the test sample and the aforementioned reference amount and, preferably, by significantly reduced amounts of glyoxylate. If the reference amount is derived from an apparently healthy subject or group of subjects with regard to diabetes or a diabetes-like condition, the drug can be identified based on the degree of identity between the amount obtained from the test sample and the aforementioned reference amount and, preferably, by an identical amount or an amount which does not differ significantly from the reference amount. A difference is, preferably, not significant and shall be characterized in that the values for the intensity are within at least the interval between 1st and 99th percentile, 5th and 95th percentile, 10th and 90th percentile, 20th and 80th percentile, 30th and 70th percentile, 40th and 60th percentile of the reference value.

The comparison is, preferably, assisted by automation. For example, a suitable computer program comprising algorithm for the comparison of two different data sets (e.g., data sets comprising the values of the characteristic feature(s)) may be used. Such computer programs and algorithm are well known in the art. Notwithstanding the above, a comparison can also be carried out manually.

The comparison may be carried out by an evaluation unit which can be part of a device for making the determination of the gyoxylate and the evaluation of the determined amount. Accordingly, such a device, preferably, comprises:
(a) an analyzing unit comprising a detection agent for glyoxylate which allows for determining the amount of glyoxylate present in the sample; and, operatively linked thereto,
(b) an evaluation unit comprising a stored reference and a data processor which allows for comparing the amount of glyoxylate determined by the analyzing unit to the stored reference, whereby a drug against diabetes can be identified.

The methods of the present invention can be implemented by the aforementioned device. A device as used herein shall comprise at least the aforementioned units. The units of the device are operatively linked to each other. How to link the units in an operating manner will depend on the type of units included into the device. For example, where means for automatically qualitatively or quantitatively determining glyoxylate are applied in an analyzing unit, the data obtained by said automatically operating unit can be processed by the evaluation unit, e.g., by a computer program which runs on a computer being the data processor in order to facilitate the diagnosis. Preferably, the units are comprised by a single device in such a case. However, the analyzing unit and the evaluation unit may also be physically separate. In such a case operative linkage can be achieved via wire and wireless connections between the units which allow for data transfer. A wireless connection may use Wireless LAN (WLAN) or the internet. Wire connections may be achieved by optical and non-optical cable connections between the units. The cables used for wire connections are, preferably, suitable for high throughput data transport.

A preferred analyzing unit for determining glyoxylate comprises a detection agent, such as an antibody, protein or aptamer which specifically recognizes glyoxylate as specified elsewhere herein, and a zone for contacting said detection agent with the sample to be tested. The detection agent may be immobilized on the zone for contacting or may be applied to said zone after the sample has been loaded. The analyzing unit shall be, preferably, adapted for qualitatively and/or quantitatively determine the amount of complexes of the detection agent and glyoxylate. It will be understand that upon binding of the detection agent to the glyoxylate, at least one measurable physical or chemical property of either glyoxylate, the detection agent or both will be altered such that the said alteration can be measured by a detector, preferably, comprised in the analyzing unit. However, where analyzing units such as test stripes are used, the detector and the analyzing units may be separate components which are brought together only for the measurement. Based on the detected alteration in the at least one measurable physical or chemical property, the analyzing unit may calculate an intensity value for glyoxylate as specified elsewhere herein. Said intensity value can then be transferred for further processing and evaluation to the evaluation unit. Analyzing units preferred according to the present invention are those which are suitable for antibody and/or enzyme based assays, such as RIA, ELISA, ECLIA, DELFIA or solid phase immune tests, or which can be used for glyoxylate determination based analytical chemistry such as capillary electrophoresis (Hubert 2001, Clinical Chemistry 47: 1319-1321) and colorimetric methods (Kyaw 1978, Clin Chim Acta 86(2):153-7) can be used.

Alternatively, an analyzing unit as referred to herein, preferably, comprises means for separating metabolites, such as chromatographic devices, and means for metabolite determination, such as spectrometry devices. Suitable devices have been described in detail above. Preferred means for compound separation to be used in the system include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or GC-MS are used in the analyzing unit.

The evaluation unit of the device, preferably, comprises a data processing device or computer which is adapted to execute rules for carrying out the comparison as specified elsewhere herein. Moreover, the evaluation unit, preferably, comprises a database with stored references. A database as used herein comprises the data collection on a suitable storage medium. Moreover, the database, preferably, further comprises a database manage-ment system. The database management system is, preferably, a network-based, hierarchical or object-oriented database management system. Furthermore, the database may be a federal or integrated database. More preferably, the database will be implemented as a distributed (federal) system, e.g. as a Client-Server-System. More preferably, the database is structured as to allow a search algorithm to compare a test data set with the data sets comprised by the data collection. Specifically, by using such an algorithm, the database can be searched for similar or identical data sets being indicative for drug against diabetes (e.g. a query search). Thus, if an identical or similar data set can be identified in the data collection, the test data set will be associated with the effect deposited for said data set, i.e. a drug effect against diabetes or no effect.

In yet a preferred embodiment of the method of the present invention, the said method further comprises the step of administering the compound suspect to be a drug against diabetes in a therapeutically effective amount to non-human animal subject prior to step a) and prior to taking the test sample, the step of taking the test sample prior to step a) and the step of killing the non-human animal subject after the test sample has been taken.

The term "therapeutically effective amount" as used herein refers to an amount of the compound which presumably should be therapeutically effective in that it potentially cures or ameliorates the disease or at least affects a symptom or clinical parameter and, preferably, the gyloxylate level. Such an amount can be either experimentally determined by using a panel of different amounts and/or may be predicted if drugs are known for a given class of compounds comprising the compound to be investigated. In some cases it might be recommended to also take into account toxicity parameters of a compound, such as EC50 or IC50 values, in order to use sub-toxic amounts of the compound when looking for a therapeutically effective amount. Details on the determination of such toxicity parameters are referred to elsewhere herein.

The test sample may be taken by any suitable technique including blood sampling, biopsy and the like. It will be understood that the sampling technique needs to be adapted for the kind of sample. However, sampling techniques for the various kinds of samples referred to herein are well known to the skilled artisan and are also referred to elsewhere herein.

The term "killing" as used herein euthanizing the non-human animal subject, preferably, after the test sample has been taken. Accordingly, the method of the present invention shall not be used to improve a health or to cure or ameliorate a disease condition in the subject. It will be understood, however, that the killing of non-human animal subject shall be made taken into consideration all due care required according to animal welfare and good laboratory practice.

Advantageously, it has been found in accordance of the present invention that glyoxylate not only is a biomarker indicating the presence of diabetes or an AGEs-related diabetic complication and, in particular, diabetic nephropathy, but also is a target for drug development. The reduction of glyoxylate has been found to be associated with a reduction of advanced glycation end products and the generation of carbonylation and other Maillard reaction related products and, thus, with an improvement of diabetes, in particular, with respect to comorbidities such as diabetic nephropathy.

Glyoxylate possesses the glycating property. The increased reactivity of glyoxylate towards production of AGEs was speculated from its molecular size and structure (Dutta et al. 2007 U. Dutta, M.A. Cohenford, M. Guha and J.A. Dain (2007) Non-enzymatic interactions of glyoxylate with lysine, arginine, and glucosamine: a study of advanced non-enzymatic glycation like compounds, Bioorg. Chem. 35: 11-24) and confirmed by the following experimental findings. Incubation of glyoxylate with lysine, arginine and glucosamine in phosphate buffer at pH 7.2 in the dark at 37°C for 3 and 30 days showed that glucosamine>lysine>>arginine were susceptible to non-enzymatic attack by glyoxylate, which resulted in a production of AGE species detectable by UV and fluorescence spectroscopy. The production of AGE species increased with the incubation time and with the increasing concentrations of lysine and glucosamine. Compared to reducing sugars such as glyceraldehyde, glucose or fructose in incubation with glucosamine, glyoxylate was 60% more reactive than glucose or fructose and 20% more reactive than glyceraldehyde. The reactivity of glyoxylate towards amino substituted lysines was also addressed through its incubation with either Nα-acetyl lysine or Nε-acetyl lysine. From the absence of any AGE species detected after these incubations it was suggested, that the lysyl groups in a protein may not be highly reactive with glyoxylate, and that glyoxylate may be more reactive with free lysine, than with lysine engaged in peptide bonds as part of a protein (Dutta loc. cit.). Indeed, glyoxylate demonstrated low (compared to glucose and methyl glyoxal), but still some overall reactivity when incubated with human serum albumin (HSA) in phosphate buffer at pH 8 for 4 weeks at 37°C: 10 mM glyoxylic acid as modifier produced approximately 10% lysine and 20% arginine side chain modifications; fibrillar state as a measure of aggregation of the glyoxylic acid-modified HSA showed small but significant enhancement, suggesting considerable changes in the protein conformation, which might cause a loss of biological activity of the modified protein (Schmitt A, Schmitt J, Munch G, Gasic-Milencovic J (2005) Characterization of advanced glycation end products for biochemical studies: side chain modifications and fluorescence characteristics. Analytical Biochemistry 338: 201-215.); small mass increase for glyoxylic acid-derived AGEs was also detected (Schmitt A, Gasic-Milenkovic J, Schmitt J (2005-2) Characterization of advanced glycation end products: Mass changes in correlation to side chain modifications. Analytical Biochemistry 346: 101-106). Reactivity of glyoxylic acid molecule towards production of AGEs is also supported by the fact, that extra-cellular production for the experimental purposes of specific AGEs, Nε-carboxymethyllysine (CML)-modified proteins, is usually accomplished through incubation of proteins just with glyoxylic acid, in the presence of the reducing agent (e.g. Valencia JV, Weldon SC, Quinn D, Kiers GH, DeGroot J, TeKoppele JM, Hughes TE (2004) Advanced glycation end product ligands for the receptor for advanced glycation end products: biochemical characterization and formation kinetics. ANALYTICAL BI-OCHEMISTRY 324 (1): 68-78., Delatour, F. Fenaille, V. Parisod, F. Arce Vera, T. Buetler (2006) Synthesis, tandem MS- and NMR-based characterization, and quantification of the carbon 13-labeled advanced glycation endproduct, 6-N-carboxymethyllysine. Amino Acids 30: 25-34). CML itself, one of the dominant AGEs, can be produced by reductive amination of glyoxylic acid with Nα-acetyl-L-lysine (Csuk, S. Stark, A. Barthel, R. Kluge, D. Ströhl (2009) A Robust Synthesis of N(epsilon)-(Carboxymethyl)-L-lysine (CML). Synthesis 2009(11): 1933-1934).

There is an urgent need to identify a new therapeutic target to prevent diabetic nephropathy, as its prevalence has been increasing worldwide (Tanaka Y, Kume S, Kitada M, Kanasaki K, Uzu T, Maegawa H, Koya D. (2012) Autophagy as a therapeutic target in diabetic nephropathy. Exp Diabetes Res. 2012; 2012:628978). Pharmacotherapy for diabetic nephropathy, which is based on the described mechanisms involved in its pathogenesis (summarised by Balakumar P, Arora MK, Ganti SS, Reddy J, Singh M. (2009) Recent advances in pharmacotherapy for diabetic nephropathy: current perspectives and future directions. Pharmacol Res. 60(1): 24-32), has never considered glyoxylate as a potential drug target. The uncovered molecular mechanism links diabetic hyperglycemia and hyperoxaluria-associated nephropathy through a small molecule glyoxylate: on the one hand it accumulates in diabetic plasma parallel to glucose, and on the other hand it is a direct precursor of oxalate, accumulation of which causes calcium oxalate uro- and nephrolithiasis. This molecular mechanism points at glyoxylate i) as the novel and unique biomarker of diabetic nephropathy and ii) as a promising anti-diabetic nephropathy drug target, as scavange of glyoxylate and lowering the amount of glyoxylate by suppressing its production shall destroy the molecular link from diabetic hyperglycemia to hyperoxaluria-induced nephropathy.

Glyoxylate is a new molecule found in accordance with the findings underlying the present invention to be consistently increased in diabetic plasma and urine. It is a common, but low-abundant and cytotoxic metabolite in a human body. Functionally, glyoxylate connects diabetic hyperglycemia and advanced glycation-related diabetic complications through its involvement into the production of advanced glycation end products (AGEs). The pathophysiological significance of glyoxylate is based in part on the observation, that metabolic detoxification of glucose derived glyoxal, which is itself a major precursors of AGEs, produce glyoxylate, and that glyoxylate possesses glycating property.

In agreement with the present findings, one aspect of renal metabolic disorders can now be reconsidered in light of dual role of glyoxylate, first, being implicated into advanced glycation, and, second, being a sole direct precursor of oxalate. Association of glyoxylate as direct metabolic oxalate precursor with kidney stone disease was extensively studied and carefully documented. Kidney stones consist of crystal-like deposits, 70-80% of which contain calcium, and the majority of calcium stones consist primarily of calcium oxalate (Coe 1992, N Engl J Med 327: 1141-1152; Johri 2010, Nephron Clinical Practice 116: C159-C171), thus making increased urinary oxalate, or hyperoxaluria, a major risk factor for kidney stone disease. The evidence, that the direct oxalate precursor glyoxylate is at the same time a product of glyoxal detoxification and a highly reactive precursor of AGE species, provides a new causal link between hyperglycemia and associated accumulation of AGEs on the one hand and oxalate induced nephropathy leading to chronic renal failure on the other hand, and suggests a direct molecular mechanism for the implication of AGEs in one of the severe diabetic comorbidities, the diabetic nephropathy, through metabolic links connecting AGEs, glyoxylate and oxalate. An integrated view of glyoxylate biochemistry points at glyoxylate being an indicator for the excess portion of glucose potentially responsible for chemical modifications, which are not controlled enzymatically (i.e. glycation) and elicites causality for development of diabetes-induced renal complications such as diabetic nephropathy. Such positioning of glyoxylate as a central molecular hub in the pathogenesis of diabetes and its complications offers multiple new options for new treatment strategies, and underlines the importance of glyoxylate as a putative target for therapeutic interventions in diabetes, diabetic nephropathy and AGE-related dysfunctions.

Moreover, advanced glycation end products (AGEs) besides of being known to accumulate at diabetes and be responsible for diabetic complications also accumulate with age and in age-related chronic diseases. In this regard, C. elegans with its longevity mutants represents a good model to study the processes associated with aging. High glucose-mediated reduction of C. elegans lifespan and involvement of glyoxylate shunt into C. elegans longevity, together with the metabolic link between glyoxylate and advanced glycation jointly point at glyoxylate as a central molecular hub underlying longevity and linking the processes of advanced glycation and aging. In accordance with these findings, metformin, a modern anti-diabetic drug, was shown to also promote lifespan in C. elegans and healthy human aging. Experimental evidence for scavenging of glyoxylate by advanced-glycation-inhibiting aminoguanidine, which shares biochemical similarity with biguanide metformin, provides a strong basis for i) glyoxylate being also scavenged by metformin and ii) involvement of glyoxylate scavenge into the mechanism of metformin action. Involvement of glyoxylate in aging through its implication into the protein damage by advanced glycation makes it a convenient biomarker of enzymatically uncontrolled aging at hyperglycemia. Additionally, scavenging of glyoxylate by one of biguanides may provide an effective control point for otherwise uncontrolled process of the premature AGE-induced aging. The method of the present invention can, thus, also be used to identify drugs which reduce damage by advanced glycation via a reduction or inactivation (by binding) of glyoxylate. These drugs are effective against diabetes but also diabetes like conditions. It will be understood that the method of the invention instead or in addition of identifying drugs against diabetes, could also be used for identifying life-promoting agents, in general.

The definitions and explanations of the terms made above apply mutatis mutandis for the following embodiments except as specified otherwise herein below.

The present invention relates to an ex vivo method for identifying a drug against diabetes comprising:
(a)contacting test cells which produce glyoxylate with a compound suspected to be a drug against diabetes for a time and under conditions which allow for said compound to interact with the cells and to affect glyoxylate production;
(b)determining the amount of available glyoxylate in said cells; and
(c) comparing the amount determined in step (b) to a reference amount for glyoxylate, whereby a compound being a drug against diabetes is identified.

The term "contacting" as used herein refers to bringing the compound suspected to be a drug against diabetes into physical contact with the said test cell. The compound shall be brought into contact for a time and under conditions sufficient to allow for interaction of the compound with its target in the cell so that the glyoxylate production or the amount of available glyoxylate can be affected. Suitable conditions and a suitable time can be selected by the skilled artisan dependent on the chemical nature of the compound. It will be understood that a compound which directly inhibits an enzyme or regulator of the glyoxylate metabolism may affect the glyoxylate production much faster than a compound which indirectly acts via inhibition of transcription or translation of such an enzyme or regulator.

The amount of available glyoxylate in the test cells can be determined as set forth elsewhere herein. Preferably, the cells of a cell culture of test cells are harvested and lysed in order to obtain a cell lysate comprising the glyoxylate. "Available glyoxylate" in accordance with the present invention refers to glyoxylate which is available for glycation reactions. Accordingly, such available glyxoxylate can be reduced by compounds, such as metformin, which act via a scavenging mechanism by binding to glyoxylate and inactivating it with respect to its glycation capabilities. In such a case, it will be understood that the total amount of glyoxylate present in a cell will not be affected by the compound. However, the amount of glyoxylate which is available for glycation reactions will be decreased due to the scavenging effect elicited by the compound.

Compounds which can be used in the method of the present invention for identifying antagonists are those which are referred to as potential antagonists elsewhere in this specification.

The term "test cells" as used herein refers to cells in a cell culture, i.e. cells which are not part of an organism. The cells shall, preferably, have an active glyoxylate metabolism and produce glyoxylate. Such active metabolism may be present endogenously in the cells or it may be implemented exogenously by, e.g., genetic engineering. Preferably, said test cells are selected from the following group of cells: hepatic HepG2 cells, Chang liver cells, rat or mice dorsal root ganglia neuronal cells, adipocytes and mesangial cells. Other cells which can be applied as test cells according to the invention encompass kidney and liver tissues of hyperoxaluric men have impaired glyoxylate metabolism (see Dean BM, Watts RWE, Westwick WJ (1967), Biochem J 105: 701-707), human HepG2 cells treated with glyoxylate (see Baker PRS, Cramer SD, Kennedy M, Assimos DG, Holmes RP (2004), Am J Physiol Cell Physiol 287: C1359-C1365; J. Knight, D.G. Assimos, L. Easter, R.P. Holmes (2010), Horm Metab Res 42: 868-873; Schnedler N, Burckhardt G, Birgitta C. Burckhardt BC (2011), J Hepatol 54:513-520) and human liver slices treated with glyoxylate (Nagata M, Ichiyama A, Takayama T, Oda T, Mugiya S, Ozono S (2009), Biomedical Research 30 (5): 295-301)

The aforementioned cells have been used to establish cell culture models to study diabetes and are, thus, preferred test cells for the method of the present invention. In particular, rat dorsal root ganglion neuron culture was developed to study the neurotoxic effects of high glucose. In dorsal root ganglion cultured in defined medium, addition of moderate glucose levels results in neurite degeneration and apoptosis. These changes are coupled with activation of caspase-3, dependent on the concentration of glucose. The apoptotic changes observed in vitro are similar to those observed in vivo; see Russell JW, Sullivan KA, Windebank AJ, Herrmann DN, Feldman EL (1999) Neurons undergo apoptosis in animal and cell culture models of diabetes. Neurobiol Dis.6(5): 347-363.

Mice primary dorsal root ganglion (DRG) neuron culture may also used as test cells accordance to the invention. Dissociated DRG cultures were developed from the adult mouse in two models of diabetes, streptozotocin (STZ)-induced type 1 diabetes and genetic type 2, db/db(-/-) mice. DRG were collected from C57BL/6J mice when they were 6-8 weeks of age. Mouse DRG were extracted from adult mice and placed in L-15 media during dissection, dissociated in papain (2 mg/ml) and 2.5% collagenase for 30 min, and then quenched in calf serum. DRG were then extracted by centrifugation, placed in warm plating medium, and triturated 20-30 times with a calf serum-coated glass pipet. Adult mouse DRG neurons were cultured in Neurobasal medium (Sigma) containing 25 mM glucose (optimal basal glucose for neurons), with 1X B27 (Sigma), 0.14 mM L-glutamine, and 40 µM FUDR to remove contaminating Schwann cells. Hyperglycemia was induced by adding 20 mM additional glucose (total 45 mM glucose) to the media; see Vincent AM, Russell JW, Sullivan KA, Backus C, Hayes JM, McLean LL, Feldman EL (2007) SOD2 protects neurons from injury in cell culture and animal models of diabetic neuropathy. Exp Neurol. 208(2): 216-227, herewith incorporated by reference, for details.

Further, a mesangial cell culture may also be used as test cells in accordance with the present invention. Rat mesangial cells cultured primarily from Sprague-Dawley rats are treated in high-glucose (HG; 50 mM) or control normal-glucose (NG; 5 mM) conditions; see Ju KD, Shin EK, Cho EJ, Yoon HB, Kim HS, Kim H, Yang J, Hwang YH, Ahn C, Oh KH (2012) Ethyl pyruvate ameliorates albuminuria and glomerular injury in the animal model of diabetic nephropathy. Am J Physiol Renal Physiol. 302(5): F606-13, details. The mesangial cell culture cells have also been reported as an in vitro model of diabetic nephropathy and could be used to investigate said comorbidity of diabetes as well. Rat mesangial cells cultured primarily from Sprague-Dawley rats are treated in high-glucose (HG; 50 mM) or control normal-glucose (NG; 5 mM) conditions (see Ju loc. cit.).

Moreover, adipocytes for assessment of glucose uptake in the basal state and after insulin stimulation have been reported and could also, preferably, be used as test cells according to the present invention. The adipose tissue is a major site of insulin action and contributes substantially to energy homeostasis. Insulin increases the extraction of glucose from circulation into adipose tissue by recruiting the glucose transporter GLUT4 to the plasma membrane. Murine 3T3-L1 adipocytes and isolated mature human adipocytes were used to test the hypothesis that dietary flavonoids may interfere with glucose transport processes. Glucose transport was assayed through kinetic characterization of 2-deoxyglucose uptake in the basal state in both cell types; Claussnitzer M, Skurk T, Hauner H, Daniel H, Rist MJ (2011) Effect of flavonoids on basal and insulin-stimulated 2-deoxyglucose uptake in adipocytes. Mol Nutr Food Res. 2011 May;55 Suppl 1:S26-34, for details.

Liver cell culture models could also preferably be applied as test cells in accordance with the present invention. Increased incidence of liver injury is often found in diabetics and hyperglycemia plays an important role in promoting liver injury through several mechanisms. The pathways through which hyperglycemia causes changes in liver of various animal models and liver cell culture models are identified, and the mechanisms and consequences of hyperglycemia induced liver injury in humans are elucidated. Some of the pathways which are hyperglycemia driven include increased oxidative and nitrosative stress, activation of stress signaling pathways and increased cytokine levels, impairment of protective mechanisms such as the expression of molecular chaperones and proteosome activity, and dysregulation of glucose and lipid metabolism. Thus, hyperglycemia induced changes in the liver's cellular environment in in vitro models have been documented extensively in the literature, clearing the way for the usage of liver cell culture models to study diabetes.

In particular, insulin resistance and type 2 diabetes have been studied in human Chang liver cells. Chang liver cells cultured to 70% confluence in RPMI1640 containing 10% fetal calf serum (FCS) were exposed for 24 h to MCBD-201 medium; or MCBD-201 containing 0.1 µM insulin and 1 mM fructose. The model was used to test the known anti-diabetic properties of a herbal plant; see Dey A, Chandrasekaran K (2009) Hyperglycemia induced changes in liver: in vivo and in vitro studies. Curr Diabetes Rev. 5(2): 67-78. Dealtry GB, Williams SI, van de Venter M, Roux S (2011) Gene regulation by Sutherlandia frutescens, a South African anti-diabetic medicinal plant, in an insulin resistant liver cell culture model of type 2 diabetes. Scientific Research and Essays, Conference proceedings, pp. 13-14, for details.

In a preferred embodiment of the aforementioned method of the invention, said reference amount for glyoxylate is derived from reference test cells which produce glyoxylate and which have not been contacted with a compound suspected to be a drug against diabetes. More preferably, an amount of glyoxylate which is decreased compared to the reference amount identifies the compound as drug against diabetes.

The test cells used for deriving the reference amount (reference cells) are, preferably, subjected to the same procedure as the test cells except that the cells shall not be contacted to the compound suspected to be a drug against diabetes, It wiii be understood that the said reference cells are of the same cell type and, preferably, cell line or, even more preferably, from the same passage and batch as the actual test cells.

Advantageously, due to the role of glyoxylate as target for the desired drug action, a cell based assay can be easily established which uses the target itself as readout. Such a cell-based assay in avoiding animal use is well suited for a preliminary screen for reducing the number of potential lead compounds, drug candidates or drugs. Moreover, the method can be automated by robotic devices and/or evaluation devices as referred to elsewhere herein.
The present invention also contemplates a method for identifying a drug against diabetes comprising the steps of the test cell-based method of the invention referred to above and subsequently the steps of the subject-based method of the present invention. In particular, the present invention, thus, contemplates a method for identifying a drug against diabetes comprising:
   (a)contacting test cells which produce glyoxylate with a compound suspected to be a drug against diabetes for a time and under conditions which allow for said compound to interact with the cells and to affect the amount of available glyoxylate;
   (b)determining the amount of available glyoxylate in said cells;
   (c) comparing the amount determined in step (b) to a reference amount for glyoxylate, whereby a compound being a drug against diabetes is first time identified or whereby a compound to be further processed in steps (d) and (e) is identified;
   (d)determining the amount of glyoxylate in a test sample of a non-human animal subject suffering from diabetes or an AGEs-related diabetic complication wherein said test sample has been taken after the subject has been brought into contact with the compound suspected to be a drug against diabetes; and
   (e)comparing the amount determined in step (a) to a reference amount for glyoxylate, whereby a compound being a drug against diabetes is second time identified or confirmed.

In a preferred embodiment of the aforementioned method of the invention, the steps of the subject-based method of the invention, i.e. steps (d) and (e) are carried out only for a compound which has been identified as a drug against diabetes according to the steps of the cell-based method of the invention, i.e. steps (a) to (c).

In another preferred embodiment of the aforementioned method, said methods may further comprise the step of determining whether a compound suspected to be a drug against diabetes is capable of binding and scavenging glyoxylate. Such a step may, preferably, be carried out prior to step (a) in order to make a pre-selection of compounds subjected to the actual method. Binding and scavenging of glyoxylate can be determined by techniques well known in the art including in vitro spectroscopy-based measurements. Preferably binding and scavenging of glyoxylate refers to an interaction between glyoxylate and drug against diabetes, which reduces or preferentially abolishes the glycating activity of glyoxylate.

Said method may further comprise the step of optimizing the drug against diabetes by testing various chemical variants of the drug for enhanced or reduced preferably enhanced binding to glyoxylate. Such a step may, preferably, be carried out after step (c) or even more preferred after step (e) in order to further optimize the drug against diabetes identified in step (c) or (d). The person skilled in the art is aware of how to design and produce the variants of the drug against diabetes to be used in this embodiment of the invention. The design of the variants is preferably based on computer based structure modelling for optimized binding to glyoxylate.

Accordingly the present invention also discloses a method for optimizing a potential drug against diabetes (independent of the steps or method mentioned before, e.g., a drug found be others means like Metformin can be improved by improved binding to glyoxylate) by testing various chemical variants of the drug for enhanced or reduced preferably enhanced binding to glyoxylate.

Advantageously, an effective screening method particularly suitable for high throughput approaches is provided according to the present invention by using the cell-based method for a pre-screening and the subject-based method for the actual screening and identification process for lead compounds, candidate drugs or drugs. By combining the two methods as specified above, only those compounds shall be investigated by the subject-based method, which have been identified already in the cell-based method. Thereby, the number of animals required in particular for large high throughput screens can be significantly reduced.

The present invention also discloses a method for the manufacture of a medicament against diabetes comprising the steps of any one of the methods of the present invention and the further step of formulating a compound identified as a drug against diabetes in a pharmaceutically acceptable form.

The term "medicament" as used herein refers, in one aspect, to a pharmaceutical composition containing the identified drug referred to above as pharmaceutical active compound, wherein the pharmaceutical composition may be used for human or non-human therapy of various diseases or disorders in a therapeutically effective dose. The identified drug, preferably, can be present in liquid or lyophilized form. The medicament is, preferably, for topical or systemic administration. Conventionally a medicament will be administered intra-muscular or, subcutaneous. However, depending on the nature and the mode of action of a compound, the medicament may be administered by other routes as well. The identified drug is the active ingredient of the composition, and is, preferably, administered in conventional dosage forms prepared by combining the drug with standard pharmaceutical carriers according to conventional procedures. These procedures may involve mixing, granulating, and compression, or dissolving the ingredients as appropriate to the desired preparation. It will be appreciated that the form and character of the pharmaceutical acceptable carrier or diluent is dictated by the amount of active ingredient with which it is to be combined, the route of administration, and other well-known variables.

A carrier must be acceptable in the sense of being compatible with the other ingredients of the formulation and being not deleterious to the recipient thereof. The pharmaceutical carrier employed may include a solid, a gel, or a liquid. Examples for solid carriers are lactose, terra alba, sucrose, talc, gelatin, agar, pectin, acacia, magnesium stearate, stearic acid and the like. Exemplary of liquid carriers are phosphate buffered saline solution, syrup, oil, water, emulsions, various types of wetting agents, and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

A diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like.

A therapeutically effective dose refers to an amount of the identified drug to be used in medicament which prevents, ameliorates or treats the symptoms accompanying a disease or condition referred to in this specification. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. The medicament referred to herein is administered at least once in order to treat or ameliorate or prevent a disease or condition recited in this specification. However, the said medicament may be administered more than one time. Specific medicaments are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent. The resulting formulations are to be adapted to the mode of administration. Dosage recommendations shall be indicated in the prescribers or users instructions in order to anticipate dose adjustments depending on the considered recipient.

The medicament may also comprise drugs in addition to the identified drug which are added to the medicament during its formulation.

Finally, it is to be understood that the formulation of a medicament takes place under GMP standardized conditions or the like in order to ensure quality, pharmaceutical security, and effectiveness of the medicament.

The invention further discloses a medicament obtainable by any of the methods of the present invention namely, the use of a drug identified by any of the methods of the present invention for the manufacture of a medicament for treating and/or preventing diabetes.

Further the invention provides, in general, a drug identified by any of the methods of the present invention for use in treating and/or preventing diabetes.

Finally, disclosed herein is a method for treating and/or preventing a patient suffering diabetes comprising administering to said patient a therapeutically effective amount of a drug identified by any of the methods of the present invention. The patient, preferably, is a human suffering from or being at risk of developing diabetes.

### FIGURES

**Figure 1** shows an overview of glyoxylate biochemistry. AGT, alanine:glyoxylate aminotransferase; AKR, aldo-keto reductase; ALDH, aldehyde dehydrogenase; GO, glycolate oxidase; FBPA, fructose-bisphosphate aldolase; GR, glyoxylate reductase; HOGA, 4-hydroxy-2-oxoglutarate aldoase; LDH, lactate dehydrogenase; PFK. phosphofructokinase

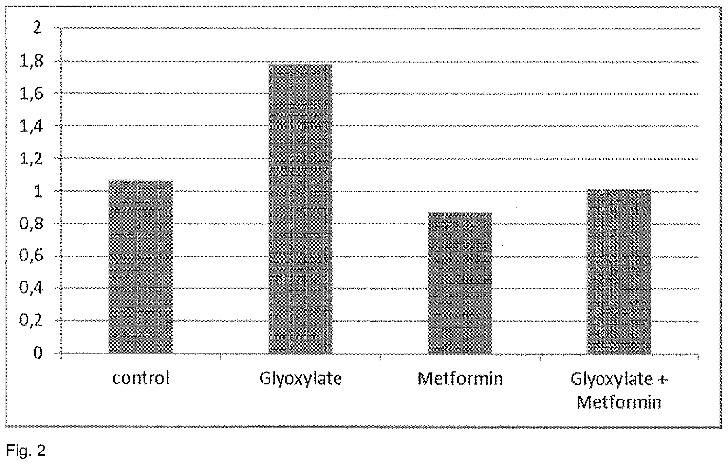

**Figure 2** shows concentrations of glyoxylate in C. elegans after treatment with 20 mM glyoxylate (Glyoxylate), 50 mM metformin (Metformin), or 20 mM glyoxylate + 50 mM metformin (Glyoxalate+Metformin). C. elegans maintained without added chemicals were used as controls. Data shown were normalized to the median of the control samples leading to ratios to control.

### EXAMPLES

The invention will now be illustrated by the following Examples.

### Example 1: Glyoxylate as drug target for diabetes drugs

Aminoguanidine, a known glyoxal scavenger (Thornalley PJ, Yurek-George A, Argirov OK (2000) Kinetics and mechanism of the reaction of aminoguanidine with the alpha-oxoaldehydes glyoxal, methylglyoxal, and 3-deoxyglucosone under physiological conditions. Biochem Pharmacol. 60(1): 55-65) was demonstrated to scavenge glyoxylate and thus divert it away from oxalate synthesis to hold promise against calcium oxalate stone disease (Berman PA, van der Watt GF, Hack DJ, Baumgarten I, (2005) Inhibition of glyoxylate conversion to oxalate in cultured human cells by the carbonyl-scavenging drug, aminoguanidine. South African J of Science 101 (5-6): 249-255). Generally, two types of carbonyl-scavenging drugs are known: Those containing thiol or amine functional groups. Examples of the thiol-containing drugs are penicillamine, cysteine, and N-acetyl-cysteine; drugs with amine functional group are represented with aminoguanidine, pyridoxamine, metformin. Such drugs have been suggested to act therapeutically in preventing hyperglycemia-induced protein damage by trapping glyoxal and methylglyoxal to form non-toxic adducts (Mehta, Lilian Wong, Peter J. O'Brien (2009) Cytoprotective mechanisms of carbonyl scavenging drugs in isolated rat hepatocytes. Chemico-Biological Interactions 178: 317-323.). The thiol-containing drugs can entrap glyoxal in the order of effectiveness: penicillamine>cysteine>N-acetyl-cysteine. For the amine-containing drugs, the order of effectiveness for glyoxal trapping was aminoguanidine>>pyridoxamine>metformin. The ability of aminoguanidine to effectively scavenge glyoxylate points at glyoxylate as a dual drug target against both advanced glycation-induced diabetic complications and hyperoxaluria-associated diabetic nephropathy.

### Example 2: Rats treated with anti-diabetic drug metformin

Two groups of each 5 male and female rats was dosed once daily with the indicated compounds with a different dose per group (see below) over 28 days.

Each dose group in the studies consisted of five rats per sex. Additional groups of each 15 male and 15 female animals served as controls. Before starting the treatment period, animals, which were 62-64 days old when supplied, were acclimatized to the housing and environmental conditions for 7 days. All animals of the animal population were kept under the same constant temperature (20-24 ± 3 °C) and the same constant humidity (30-70 %). The animals of the animal population were fed ad libitum. The food to be used was essentially free of chemical or microbial contaminants. Drinking water was also offered ad libitum. Accordingly, the water was free of chemical and microbial contaminants as laid down in the European Drinking Water Directive 98/83/EG. The illumination period was 12 hours light followed by 12 hours darkness (12 hours light, from 6:00 to 18:00, and 12 hours darkness, from 18:00 to 6:00). The studies were performed in an AAALAC-approved laboratory in accordance with the German Animal Welfare Act and the European Council Directive 86/609/EE. The test system was arranged according to the OECD 407 guideline for the testing of chemicals for repeated dose 28-day oral toxicity study in rodents. The test substance was dosed and administered as follows:
Metformin hydrochloride was administered by gavage (high dose group at 1 g/kg body weight, low dose group at 0.2 g/kg body weight), in drinking water containing 0.5% Carboxymethyl cellulose (Tylose CB30000) (administration volume: 10 ml/kg body weight).

In the morning of day 7, 14, and 28, blood was taken from the retroorbital venous plexus from fasted anaesthetized animals. From each animal, 1 ml of blood was collected with EDTA as anticoagulant. The samples were centrifuged for generation of plasma. All plasma samples were covered with a N₂ atmosphere and then stored at -80°C until analysis.

For mass spectrometry-based metabolite profiling analyses plasma samples were extracted and a polar and a non-polar (lipid) fraction was obtained. For GC-MS analysis, the non-polar fraction was treated with methanol under acidic conditions to yield the fatty acid methyl esters. Both fractions were further derivatised with O-methyl-hydroxyamine hydrochloride and pyridine to convert Oxo-groups to O-methyloximes and subsequently with a silylating agent before analysis.

Following comprehensive analytical validation steps, the data for each analyte were normalized against data from pool samples. These samples were run in parallel through the whole process to account for process variability. The significance of treatment group values specific for sex, treatment duration and metabolite was determined by comparing means of the treated groups to the means of the respective untreated control groups using WELCH-test and quantified with treatment ratios versus control and p-values.

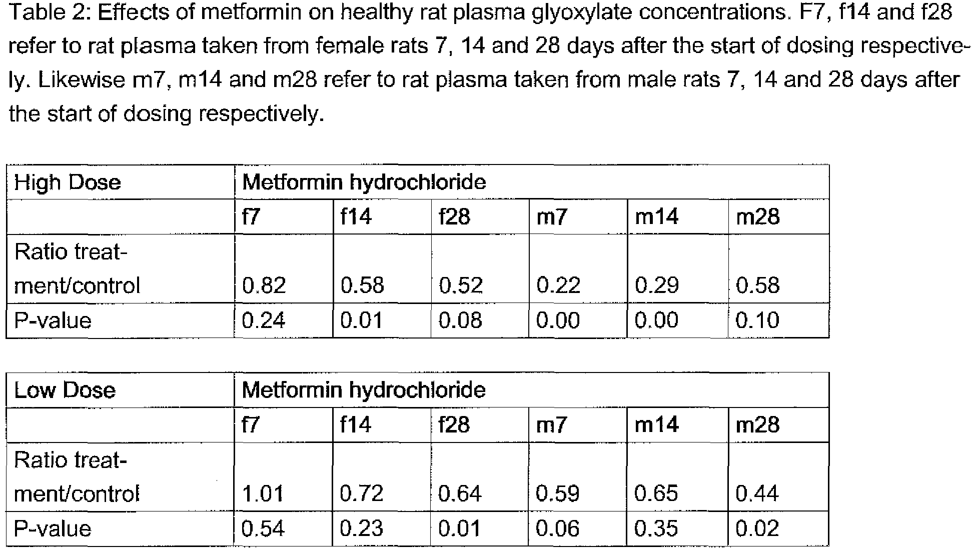

As is evident from the above Table 2, metformin is capable of strongly reducing glyoxylate concentrations found in a rat model system.

### Example 3: Detection of Glyoxylate in extracts from C. elegans

Nematodes were harvested and extensively washed with buffer and the resulting pellet was disrupted using glass beats in combination with mechanical force. Samples were extracted with methyl-tert-butylether, methanol and extraction buffer and sonicated for 15 minutes. 50 % of a mixture of water/methanol (3:1) was added, vigorously mixed and centrifuged. The polar phase was evaporated to dryness. After addition of water and a mixture of ethanol and dichlormethan, the sample was fractioned into an aqueous, polar phase and an organic, lipophilic phase. 13C-glyoxylate was added as internal standard and the extracts were derivatized in the following way: The methoximation of the carbonyl groups was carried out by reaction with methoxyamine hydrochloride (20 mg/mL in pyridine, 50 µL for 1.5 hours at 60°C) in a tightly sealed vessel. Finally, the derivatization with 50 µL of N-methyl-N-(trimethylsilyl)-2,2,2-trifluoroacetamide (MSTFA) was carried out for 30 minutes at 60°C, again in the tightly sealed vessel. The final volume before injection into the GC was 100 µL. The GC-MS systems consist of an Agilent 6890 GC coupled to an Agilent 5973 MSD. Autosamplers were CompiPal or GCPal from CTC.

For the analysis a usual commercial capillary separation column (30 m x 0,25 mm x 0,5 µm) with an arylene/poly-methyl-siloxane stationary phases was used (for example: DB-XLB, Agilent Technologies). 0.5 µL of the final volume was injected splitless and the oven temperature program was started at 70 °C and ended at 320 °C with different heating rates starting with low rates in order to achieve a sufficient chromatographic separation and number of scans for glyoxylate analysis and changing to a high heating rate after glyoxylate elution. Furthermore usual GC-MS standard conditions, for example constant flow with nominal 1 to 1.7 mL/min. and helium as the mobile phase gas and standard tune conditions were applied. Ionization was done by electron impact with 70 eV, scanning 2 characteristic mass fragments of the glyoxylate derivate and its internal standard, respectively within an appropriate time window. Scan rate was 25 scans/sec. Glyoxylate values were normalized to Total Ion Count of each sample to adjust individual deviation of sample volume. Furthermore, the data were normalized to the median of the control samples leading to ratios to control.

### Example 4:

We tested the hypothesis that metformin suppresses glyoxylate concentration in in vivo experiments with the model worm Caenorhabditis elegans, by means of its treatment with glyoxylate and metformin separately and in combination of glyoxylate plus metformin.

C. elegans culture was maintained as described in Soukup et al. (Soukup et al. (2012) Formation of Phosphoglycosides in Caenorhabditis elegans: A Novel Biotransformation Pathway. PLoS One. 2012;7(10):e46914) and Geillinger et al. (Geillinger et al. (2012) Dynamic Changes of the Caenorhabditis elegans Proteome during Ontogenesis Assessed by Quantitative Analysis with 15N Metabolic Labeling. J. Proteome Res. 11(9): 4594). To observe significant results, each treatment group of C. elegans consisted of 5 replicated batches, each of 50-100 µl worm pellet. The experiment included four treatment groups:
1) C. elegans, wild type (wt) - on growth medium (no treatment, control).
2) C. elegans, wt - on growth medium plus 50 mM metformin (methodology for Metformin application and doses as described in Onken & Driscoll (Onken B, Driscoll M (2010) Metformin induces a dietary restriction-like state and the oxidative stress response to extend C. elegans healthspan via AMPK, LKB1, and SKN-1. PLoS ONE 5(1): e8758.).
3) C. elegans, wt - on growth medium plus 20 mM glyoxylate.
4) C. elegans, wt - on growth medium plus 20 mM glyoxylate and 50 mM metformin.

After treatment, C. elegans was washed, sampled, metabolite extraction was carried out, and glyoxylate level was detected as described in Example 3. From the GC-MS metabolite profiles, total ion count (TIC) as a measure of the absolute intensity value was calculated for each batch. Measured glyoxylate levels were then volume-normalized, using TICs.

Results are summarized in Fig. 2 and in Table 3.

**Table 3: Ratios of glyoxylate concentrations in C. elegans after the indicated treatments**

| | Ratio | TTest |
|---|---|---|
| Glyoxylate / control | 1.81 | 0.0003 |
| Glyoxylate + Metformin / control | 1.02 | 0.4861 |
| Metformin/ control | 0.88 | 0.0477 |

It was found that
1) Wt C. elegans contained native glyoxylate.
2) Treatment with metformin significantly decreased native glyoxylate levels in C. elegans.
3) Treatment of C. elegans with glyoxylate led to significant increase of endogenous glyoxylate.
4) Treatment with metformin simultaneously to treatment with glyoxylate overwrote the increase of endogenous glyoxylate and restored the native glyoxylate level observed in group (1).

### Example 5: Glyoxylate is increased in the plasma of diabetes patients

Subjects were selected a prospective study from a total of 789 participants that volunteered to participate in an oral glucose tolerance test (OGTT) assessment. Prior to selection participants were grouped according to their fasting plasma glucose (prior to the OGTT) and according to their OGTT categorization.

Standard WHO diabetes categories were applied (WHO 2006):

| | |
|---|---|
| Diabetes: | FPG ≥7.0 mmol/L or 2HPG ≥ 11.1 mmol/L; |
| IGT: | FPG <7.0mmol/L and 2HPG ≥ 7.8 and < 11.1 mmol/L; |
| IFG: | FPG 6.1 to 6.9 mmol/L and 2HPG < 7.8mmol/L, |
| Healthy: | FPG ≤ 6.0 mmol/L and 2HPG < 7.8 mmol/L). |

| | |
|---|---|
| 2HPG = plasma 2h after standardized 75g oral glucose challenge | |

Selection was performed for best matching of the diabetes categories as well as potential confounders such as center, gender, body mass index and age. Finally, 478 study participants were included into the prospective study part.

**Table 4A: Diabetes categories after measurements of fasting plasma glucose and OGTT assessment for the 478 participants in the prospective study part**

| Diabetics identified only through 2HPG not through FPG | Diabetics identified only or additionally through FPG | IFG+IGT | IGT | IFG Healthy | |
|---|---|---|---|---|---|
| 28 | 30 | 77 | 39 | 127 | 177 |

**Table 4B: Subset of above described subjects measured with the SIM method at OGTT 120**

| Diabetics identified only through 2HPG not through FPG | Diabetics identified only or additionally through FPG | IFG+IGT | IGT | IFG | Healthy |
|---|---|---|---|---|---|
| 23 | 23 | 55 | 36 | 98 | 50 |

Metabolite profiling was performed on the fasted plasma samples obtained from study participants directly prior to the OGTT, as well as on plasma samples 120 min after standard oral glucose bolus (75 g). Plasma was processed by standard protocols and separated from blood within approximately 60 min. Plasma samples were immediately frozen and stored at -80°C. Transport of samples from sampling site to site of biochemical analysis was on dry ice.

It was found that glyoxylate is a predictor for diabetes (ratio of glyoxylate concentration in diabetic vs. healthy subjects 1.23 (p-value 0.011), with glyoxylate concentration being increased in the plasma of diabetic subjects. It was further found that the increase of glyoxylate concentrations is further aggravated in diabetic subjects by glucose challenge (ratio 1.72, p-value 0.0043).

### Example 6: Detection of Glyoxylate in Human plasma

Proteins were separated by precipitation from blood plasma. After addition of water and a mixture of ethanol and dichlormethane the remaining sample was fractioned into an aqueous, polar phase and an organic, lipophilic phase.

For the polar phase the derivatization was performed in the following way: The methoximation of the carbonyl groups was carried out by reaction with methoxyamine hydrochloride (20 mg/ml in pyridine, 50 l for 1.5 hours at 60°C) in a tightly sealed vessel. 10 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 µl of N-methyl-N-(trimethylsilyl)-2,2,2-trifluoroacetamide (MSTFA) was carried out for 30 minutes at 60°C, again in the tightly sealed vessel. The final volume before injection into the GC was 100 µl. The GC-MS systems consist of an Agilent 6890 GC coupled to an Agilent 5973 MSD. Autosamplers were CompiPal or GCPal from CTC.

For the analysis usual commercial capillary separation columns (30 m x 0,25 mm x 0,25 µm) with different poly-methyl-siloxane stationary phases containing 0% to 35% of aromatic moieties, depending on the analyzed sample materials and fractions from the phase separation step, were used (for example: DB-1 ms, HP-5ms, DB-XLB, DB-35ms, Agilent Technologies). Up to 1 µL of the final volume was injected splitless and the oven temperature program was started at 70 °C and ended at 340 °C with different heating rates depending on the sample material and fraction from the phase separation step in order to achieve a sufficient chromatographic separation and number of scans within each analyte peak. Furthermore RTL (Retention Time Locking, Agilent Technologies) was used for the analysis and usual GC-MS standard conditions, for example constant flow with nominal 1 to 1.7 ml/min. and helium as the mobile phase gas and standard tune conditions were applied. Ionisation was done by electron impact with 70 eV, scanning characteristic mass fragments of each analyte within an appropriate time window that consisted of 3 ion masses.

### Example 7: Determination of Glyoxylate levels in plasma of diabetic db/db-/- mutant mice

To further explore potential tissue sources of increased plasma glyoxylate levels in diabetes of humans and in animal models, the ob/ob-/- and the db/db-/- mutant mouse models are examined and metabolite levels are measured. The ob/ob-/- and the db/db-/- mouse models are well described for obesity and diabetes, respectively.

Whereas the ob/ob-/- mouse has a deficiency in leptin production, the db/db-/- mouse has a deficient leptin receptor activity. Consequently, both models have defects in leptin signaling, are highly hyperphagic and develop obesity and insulin resistance. Importantly, their difference in genetic background (e.g. C57BL/6J and C57BL/KsJ for ob/ob-/- and db/db-/- mouse, respectively) results in pancreatic hypertrophy in the ob/ob-/- mouse model and a pancreatic atrophy in db/db-/- mouse model.

The db/db-/- diabetes mouse is studied in comparison to the ob/ob-/- mouse, which serves as an obesity model. Metabolite profiling is performed as described before. Metabolite profiling results of db/db-/- and ob/ob-/- are analyzed against their respective control groups. Metabolite profiling results of db/db-/- and ob/ob-/- are also compared and analyzed statistically after normalization with respective control groups.

An increase in plasma glucose for db/db-/- mice as compared to control animals is observed. Similarly, metabolite profiling data show an increase in plasma glyoxylate levels in the db/db-/- mouse model, compared to its control. Thus, similar to plasma from untreated diabetic human subjects, plasma from the db/db-/- mouse model shows high concentrations of glyoxylate and this correlates with glucose.

## Claims

1. A method for identifying a drug against diabetes comprising:
(a) determining the amount of glyoxylate in a test sample of a non-human animal subject suffering from diabetes or advanced glycation end products (AGEs) related diabetic complications wherein said test sample has been taken after the subject has been brought into contact with a compound suspected to be a drug against diabetes; and
(b) comparing the amount determined in step (a) to a reference amount for glyoxylate, whereby a compound being a drug against diabetes is identified.

2. The method of claim 1, wherein said reference amount for glyoxylate is derived from a subject or group of subjects suffering from diabetes or AGEs-related diabetic complications wherein said subject(s) have not been brought into contact with the compound suspected to be a drug against diabetes.

3. The method of claim 1, wherein said reference amount is the amount of glyoxylate present in a reference sample which has been taken prior to contacting the subject to the compound suspected to be a drug against diabetes.

4. The method of claim 2 or 3, wherein an identical or increased amount of glyoxylate in the test sample in comparison to the reference amount identifies a compound not being a drug against diabetes, whereas a decreased amount of glyoxylate in the test sample in comparison to the reference amount identifies a compound being a drug against diabetes.

5. The method of claim 1, wherein said reference amount for glyoxylate is derived from an apparently healthy subject or group of subjects with regard to diabetes or AGEs-related diabetic complications.

6. The method of claim 5, wherein an increased amount of glyoxylate in the test sample in comparison to the reference amount identifies a compound not being a drug against diabetes, whereas an identical or decreased amount of glyoxylate in the test sample in comparison to the reference amount identifies a compound being a drug against diabetes.

7. The method of any one of claims 1 to 6, wherein said subject is a rodent, preferably a mouse or a rat, a pig, a rabbit, a cat, a dog, or a nematode.

8. The method of any one of claim 1 to 7, wherein the method further comprises:
(i) the step of administering the compound suspect to be a drug against diabetes in a therapeutically effective amount to the subject prior to step a) and prior to taking the test sample,
(ii) the step of taking the test sample prior to step a); and
(iii) the step of sacrificing the subject after the test sample has been taken.

9. The method of any one of claims 1 to 8, wherein said test sample is a sample of a body fluid of the subject.

10. An ex vivo method for identifying a drug against diabetes comprising:
(a) contacting test cells which produce glyoxylate with a compound suspected to be a drug against diabetes for a time and under conditions which allow for said compound to interact with the cells and to affect the amount of available glyoxylate;
(b) determining the amount of glyoxylate in said cells; and
(c) comparing the amount determined in step (b) to a reference amount for glyoxylate, whereby a compound being a drug against diabetes is identified.

11. The method of claim 10, wherein said reference amount for glyoxylate is derived from reference test cells which produce glyoxylate and which have not been contacted with a compound suspected to be a drug against diabetes.

12. The method of claim 10 or 11, wherein an amount of glyoxylate which is decreased compared to the reference amount identifies the compound as drug against diabetes.

13. The method of any one of claims 10 to 12, wherein said test cells are selected from the following group of cells: hepatic HepG2 cells, Chang liver cells, rat or mice dorsal root ganglia neuronal cells, adipocytes and mesangial cells.

14. A method for identifying a drug against diabetes comprising the steps of the method of any one of claims 10 to 13 and subsequently the steps of the method of any one of claims 1 to 9.

15. The method of claim 14, wherein the steps of the method of any one of claims 1 to 9 are carried out only for a compound which has been identified as a drug against diabetes according to the steps of the method of any one of claims 10 to 13.

## Patentansprüche

1. Verfahren zum Identifizieren eines Arzneimittels gegen Diabetes, umfassend:
(a) Bestimmen der Menge an Glyoxylat in einer Testprobe eines nichtmenschlichen Tiersubjekts, das an Diabetes oder mit fortgeschrittenen Glycosylierungs-Endprodukten (AGEs) verbundenen diabetischen Komplikationen leidet, wobei die Testprobe genommen wurde, nachdem das Subjekt mit einer Verbindung, die mutmaßlich ein Arzneimittel gegen Diabetes ist, in Kontakt gebracht worden ist; und
(b) Vergleichen der bei Schritt (a) bestimmten Menge mit einer Referenzmenge für Glyoxylat, wodurch eine Verbindung als Arzneimittel gegen Diabetes identifiziert wird.

2. Verfahren gemäß Anspruch 1, wobei die Referenzmenge für Glyoxylat aus einem Subjekt oder einer Gruppe von Subjekten stammt, die an Diabetes oder mit AGEs verbundenen diabetischen Komplikationen leiden, wobei das Subjekt/die Subjekte nicht mit der Verbindung, die mutmaßlich ein Arzneimittel gegen Diabetes ist, in Kontakt gebracht worden ist/sind.

3. Verfahren gemäß Anspruch 1, wobei die Referenzmenge die Menge an Glyoxylat ist, die in einer Referenzprobe vorhanden ist, die genommen wurde, bevor das Subjekt mit der Verbindung, die mutmaßlich ein Arzneimittel gegen Diabetes ist, in Kontakt gebracht worden ist.

4. Verfahren gemäß Anspruch 2 oder 3, wobei eine gleiche oder erhöhte Menge an Glyoxylat in der Testprobe im Vergleich zu der Referenzmenge eine Verbindung identifiziert, die kein Arzneimittel gegen Diabetes ist, während eine erniedrigte Menge an Glyoxylat in der Testprobe im Vergleich zu der Referenzmenge eine Verbindung identifiziert, die ein Arzneimittel gegen Diabetes ist.

5. Verfahren gemäß Anspruch 1, wobei die Referenzmenge für Glyoxylat aus einem hinsichtlich Diabetes oder mit AGEs verbundenen diabetischen Komplikationen offensichtlich gesunden Subjekt oder Gruppe von Subjekten stammt.

6. Verfahren gemäß Anspruch 5, wobei eine erhöhte Menge an Glyoxylat in der Testprobe im Vergleich zu der Referenzmenge eine Verbindung identifiziert, die kein Arzneimittel gegen Diabetes ist, während eine gleiche oder erniedrigte Menge an Glyoxylat in der Testprobe im Vergleich zu der Referenzmenge eine Verbindung identifiziert, die ein Arzneimittel gegen Diabetes ist.

7. Verfahren gemäß einem der Ansprüche 1 bis 6, wobei das Subjekt ein Nager, vorzugsweise eine Maus oder eine Ratte, ein Schwein, ein Kaninchen, eine Katze, ein Hund oder ein Fadenwurm ist.

8. Verfahren gemäß einem der Ansprüche 1 bis 7, wobei das Verfahren ferner umfasst:
(i) den Schritt des Verabreichens der Verbindung, die mutmaßlich ein Arzneimittel gegen Diabetes ist, in einer therapeutisch wirksamen Menge an das Subjekt vor Schritt a) und vor dem Nehmen der Testprobe,
(ii) den Schritt des Nehmens der Testprobe vor Schritt a); und
(iii) den Schritt des Tötens des Subjektes, nachdem die Testprobe genommen worden ist.

9. Verfahren gemäß einem der Ansprüche 1 bis 8, wobei die Testprobe eine Probe eines Körperfluids des Subjektes ist.

10. *Ex-vivo*-Verfahren zum Identifizieren eines Arzneimittels gegen Diabetes, umfassend:
(a) Inkontaktbringen von Testzellen, die Glyoxylat erzeugen, mit einer Verbindung, die mutmaßlich ein Arzneimittel gegen Diabetes ist, für einen Zeitraum und unter Bedingungen, die erlauben, dass die Verbindung mit den Zellen wechselwirkt und die Menge an verfügbarem Glyoxylat beeinflusst;
(b) Bestimmen der Menge an Glyoxylat in den Zellen; und
(c) Vergleichen der bei Schritt (b) bestimmten Menge mit einer Referenzmenge für Glyoxylat, wodurch die Verbindung als Arzneimittel gegen Diabetes identifiziert wird.

11. Verfahren gemäß Anspruch 10, wobei die Referenzmenge für Glyoxylat aus Referenz-Testzellen stammt, die Glyoxylat herstellen und die nicht mit einer Verbindung, die mutmaßlich ein Arzneimittel gegen Diabetes ist, in Kontakt gebracht worden sind.

12. Verfahren gemäß Anspruch 10 oder 11, wobei eine Menge an Glyoxylat, die im Vergleich zu der Referenzmenge erniedrigt ist, die Verbindung als Arzneimittel gegen Diabetes identifiziert.

13. Verfahren gemäß einem der Ansprüche 10 bis 12, wobei die Testzellen ausgewählt sind aus der folgenden Gruppe von Zellen: Leber-HepG2-Zellen, Chang-Leberzellen, Dorsalwurzelganglien-Nervenzellen von Ratten oder Mäusen, Adipozyten und Mesangialzellen.

14. Verfahren zum Identifizieren eines Arzneimittels gegen Diabetes, umfassend die Schritte gemäß dem Verfahren gemäß einem der Ansprüche 10 bis 13 und nachfolgend die Schritte des Verfahrens gemäß einem der Ansprüche 1 bis 9.

15. Verfahren gemäß Anspruch 14, wobei die Schritte des Verfahrens gemäß einem der Ansprüche 1 bis 9 nur für eine Verbindung durchgeführt werden, die gemäß den Schritten des Verfahrens gemäß einem der Ansprüche 10 bis 13 als Arzneimittel gegen Diabetes identifiziert worden ist.

## Revendications

1. Procédé d'identification d'un médicament contre le diabète comprenant les étapes de :
a) détermination de la quantité de glyoxylate dans un échantillon de test d'un animal non-humain souffrant de diabète ou des produits terminaux de glycation avancée (AGE) liés aux complications du diabète, ledit échantillon de test ayant été prélevé après que le sujet ait été mis en contact avec un composé présumé être un médicament contre le diabète ; et
b) comparaison de la quantité déterminée à l'étape a) avec une quantité de référence pour le glyoxylate, où on identifie un composant comme étant un médicament contre le diabète.

2. Procédé selon la revendication 1, ladite quantité de référence pour le glyoxylate étant dérivée d'un sujet ou d'un groupe de sujets souffrant de diabète ou de complications du diabète liées aux AGE, ledit ou lesdits sujets n'ayant pas été mis en contact avec le composé présumé être un médicament contre le diabète.

3. Procédé selon la revendication 1, ladite quantité de référence étant la quantité de glyoxylate présente dans un échantillon de référence qui a été prélevée avant mise en contact du sujet avec le composé présumé être un médicament contre le diabète.

4. Procédé selon la revendication 2 ou 3, dans lequel une quantité identique ou accrue de glyoxylate dans l'échantillon de test par rapport à la quantité de référence identifie un composé qui n'est pas un médicament contre le diabète, alors qu'une quantité réduite de glyoxylate dans l'échantillon de test par rapport à la quantité de référence identifie un composé comme médicament contre le diabète.

5. Procédé selon la revendication 1, ladite quantité de glyoxylate étant dérivée d'un sujet ou d'un groupe de sujets apparemment sain concernant le diabète ou les complications du diabète liées aux AGE.

6. Procédé selon la revendication 5, dans lequel une quantité accrue de glyoxylate par rapport à la quantité de référence identifie un composé qui n'est pas un médicament contre le diabète, alors qu'une quantité réduite ou identique de glyoxylate dans l'échantillon de test par rapport à la quantité de référence identifie un composé médicament contre le diabète.

7. Procédé selon l'une quelconque des revendications 1 à 6, dans lequel ledit sujet est un rongeur, de préférence une souris ou un rat, un cochon, un lapin, un chat, un chien ou un nématode.

8. Procédé selon l'une quelconque des revendications 1 à 7, ledit procédé comprenant en outre :
i) l'étape d'administration du composé présumé être un médicament contre le diabète dans une quantité thérapeutiquement efficace au sujet avant l'étape a) et avant prélèvement de l'échantillon de test ;
ii) l'étape de prélèvement de l'échantillon de test avant l'étape a) ; et
iii) l'étape de sacrifice du sujet après prélèvement de l'échantillon de test.

9. Procédé selon l'une quelconque des revendications 1 à 8, dans lequel ledit échantillon de test est un échantillon de fluide corporel du sujet.

10. Procédé *ex vivo* d'identification d'un médicament contre le diabète, comprenant :
a) la mise en contact de cellules de test qui produisent du glyoxylate avec un composé présumé être un médicament contre le diabète pendant une durée et dans des conditions qui permettent audit composé d'interagir avec les cellules et d'affecter la quantité de glyoxylate disponible ;
b) la détermination de la quantité de glyoxylate dans lesdites cellules ; et
c) la comparaison de la quantité déterminée à l'étape b) avec une quantité de référence pour le glyoxylate, où est identifié un composé comme étant un médicament contre le diabète.

11. Procédé selon la revendication 10, dans lequel ladite quantité de référence de glyoxylate est dérivée de cellules de test de référence qui produisent du glyoxylate et qui n'ont pas été mises en contact avec un composé présumé être un médicament contre le diabète.

12. Procédé selon la revendication 10 ou 11, dans lequel une quantité réduite de glyoxylate par rapport à la quantité de référence identifie le composé comme médicament contre le diabète.

13. Procédé selon l'une quelconque des revendications 10 à 12, dans lequel lesdites cellules de test sont choisies dans le groupe de cellules suivant : cellules hépatiques HepG2, cellules hépatiques Chang, cellules neuronales de ganglion spinal de rat ou de souris, adipocytes et cellules mésangiales.

14. Procédé d'identification d'un médicament contre le diabète comprenant les étapes de procédé selon l'une quelconque des revendication 10 à 13, puis les étapes du procédé selon l'une quelconque des revendications 1 à 9.

15. Procédé selon la revendication 14, dans lequel les étapes du procédé selon l'une quelconque des revendications 1 à 9 ne sont effectuées que pour un composé qui a été identifié comme un médicament contre le diabète selon les étapes de l'une quelconque des revendication 10 à 13.
